# EUROPEAN PATENT SPECIFICATION

(11) **EP 3 311 373 B1**
(45) Date of publication and mention of the grant of the patent: **22.01.2025**
(21) Application number: 15739071.7
(22) Date of filing: 22.06.2015
(51) Int. Cl.: G09B 23/28

(54) **DEVICES AND METHODS FOR DRUG ADMINISTRATION AND MIXING, AND TRAINING OF PROPER TECHNIQUES THEREFOR**
VORRICHTUNGEN UND VERFAHREN ZUR ARZNEIMITTELVERABREICHUNG UND -MISCHUNG SOWIE TRAINING DER KORREKTEN TECHNIKEN DAFÜR
DISPOSITIFS ET PROCÉDÉS D'ADMINISTRATION ET DE MÉLANGE DE MÉDICAMENTS, ET D'APPRENTISSAGE DE TECHNIQUES APPROPRIÉES À CET EFFET

(30) Priority: 19.06.2015 US 201562182426 P
(43) Date of publication of application: 25.04.2018
(73) Proprietor: Janssen Pharmaceutica, N.V., 2340 Beerse (BE)
(72) Inventor: SCRIMGEOUR, Ian, Dunbar EH42 1LT (GB); MARTIN, Scott, Edinburgh EH6 4ED (GB); MCLUSKY, James, Edinburgh EH6 4TF (GB); GLENCROSS, James, Edinburgh EH6 7JG (GB); HUTTON, Blair, Edinburgh EH7 4FR (GB); FOLEY, Nick, Edinburgh EH3 5PE (GB); BURÓN VIDAL, José Antonio, Parede 2775-343 (PT); KRULEVITCH, Peter, A., Pleasanton, California 94566 (US)
(74) Representative: Carpmaels & Ransford LLP
(86) International application number: PCT/US2015/036969
(87) International publication number: WO 2016/204795

(56) References cited:
- US-A1- 2008 015 503
- US-B1- 9 022 988

## Description

### Background

Pharmaceutical products intended for delivery by injection may be stored in vials or prefilled syringes. 1n either case, when such products consist of two or more liquid and/or solid phases, they must be agitated prior to administration for optimum result, usually by manual shaking. Health Care Professionals (HCPs) and in some cases patients or caregivers, may not provide consistent agitation for a number of reasons. They may not be familiar with the pharmaceutical product; they may have habituated experience of similar pharmaceutical products which they presume to have the same or similar preparation steps; or they may mis-time the shake duration or required shaking vigor, mistakenly underestimating the time or vigor required to properly agitate the device and adequately mix the product.

US9022988B1 describes a cartridge-based computer controlled self-injection device which automatically regulates time and dosage administered to a patient. Cartridges can be electronically tagged so the injection device can determine the content, volume, expiration date, and physician prescribed dosage timetable. Authentication verifies the device and ensures the proper patient is using the device with the appropriate therapeutic injectant. Authentication further provides information controlling device operation including expiration dates and clock synchronization. Therapeutic injectant administration statistics, such as date and time of administration of a therapeutic injectant are stored and provide to a health care provider for monitoring. Patients can be notified of nearing or missed administrations.

### Summary of the Disclosure

We have identified a need for a device that would provide the HCP with the knowledge and experience of the minimum duration and vigor of shaking required for the preparation of various pharmaceutical products such as, for example, INVEGA TRINZA^{™}, which consists of particles in a suspension. We have also identified a need for a device that alerts the user when too much time has expired after mixing and the mixing step must be repeated. Yet another need that we have identified is to ensure that the device only works with the specified syringe for a particular product, and not, for example, a competitor syringe. This ensures that people do not mistakenly use the device with the wrong drug product, and can also be used as a means for differentiating one product relative to a competitor.

Accordingly, we have devised various embodiments of devices and methods to operate such devices to fulfill the needs or mitigate the shortcomings that we have identified above. In one aspect of the invention, we have devised a device for training users how to properly mix pharmaceutical components. In another aspect, we have devised a device for mixing and assisting with the administration of properly mixed pharmaceutical components. In another aspect, we have devised a device for mixing and administrating properly mixed pharmaceutical components. In another aspect, we have devised a device for attachment to a pharmaceutical delivery device and for assisting with the administration of properly mixed pharmaceutical components. All of these devices are referenced herein (in both the description and claims) as the "device". The device includes a housing that extends along a longitudinal axis with a power source disposed in the housing and a microcontroller disposed in the housing and electrically powered by the power source as well as a user notification device and an accelerometer disposed in the housing and electrically connected to the microcontroller. In this device, the microcontroller is configured to detect a motion and orientation of the housing and indicate via the user notification device as to whether the motion or orientation of the housing being shaken during one of a drug administration or a training event is sufficient with respect to predetermined thresholds including magnitude of the force applied during the shaking, the orientation of the housing and duration of such shaking.

Another aspect of the invention includes a method of operating such a device. The method can be achieved by determining from the accelerometer if the magnitude of the motion and orientation of the housing are sufficient with respect to predetermined thresholds including magnitude of the force applied during the shaking, the orientation of the housing and duration of such shaking; and announcing via the user notification device as to whether the motion or orientation of the housing being shaken during one of a drug administration or a training event meets the predetermined thresholds.

In addition to the various aspects described above, other features recited below can be utilized in conjunction therewith to arrive at different permutations of the invention. For example, the device may include a start switch electrically connected to the microcontroller; the accelerometer may include a 3-axis accelerometer; the accelerometer is configured to activate the microcontroller upon detection of movements of the housing during one of a drug administration or a training event; the microcontroller is configured to detect when shaking of the housing has ended prematurely, or if the level of shaking vigor has reduced to a level below the pre-set threshold, to enter a pause mode to allow the user to restart the shaking during one of a drug administration or a training event; the microcontroller is configured to set a timer and determine when a maximum allowable time after shaking of the housing has elapsed to warn the user to shake the device again during one of a drug administration or a training event; the housing may include a syringe barrel element with finger flange and one end and a barrel tip spaced apart along the longitudinal axis; the housing may include a body with a slot sized to accept a syringe barrel; the housing may include a housing provided with a compartment and a lid to receive an entire syringe; the housing may include an elongated body approximately the same length as a syringe barrel such that a syringe is inserted into a syringe receiving hole in the body and retained by the compressive force applied by the finger-like members between a syringe finger flange and a body base; the housing may include a body with a syringe accepting slot sized to accept a syringe barrel; the housing may include a puck-like body with a syringe accepting hole so that in use a syringe is inserted into the syringe accepting hole and is held in place with a user's thumb on an underside thumb grip.

These and other embodiments, features and advantages will become apparent to those skilled in the art when taken with reference to the following more detailed description of the exemplary embodiments of the invention in conjunction with the accompanying drawings that are first briefly described.

### Brief Description of the Figures

The foregoing and other features and advantages of the invention will be apparent from the following, more particular description of preferred embodiments of the invention, as illustrated in the accompanying drawings.
Fig. 1A is a block diagram schematic of the electronic system;
Figure 1B illustrates a standalone mimic trainer embodiment, general view;
Figure 2 illustrates a standalone mimic trainer embodiment, orthographic views;
Figure 3 illustrates a syringe barrel attachment embodiment, showing device separate from, and attached to syringe;
Figure 4 illustrates a syringe barrel attachment embodiment, front, left and right side;
Figure 5 illustrates a syringe barrel attachment embodiment, front, top and bottom side;
Figure 6 illustrates a syringe case embodiment, general view showing syringe next to open case;
Figure 7 illustrates a syringe case embodiment, general view showing syringe inside open case;
Figure 8 illustrates a syringe case embodiment, general view showing closed case;
Figure 9 illustrates a syringe case embodiment, general view showing front, top and side view;
Figure 10 illustrates a syringe pot embodiment with syringe inside, showing front and side views;
Figure 11 illustrates a syringe pot embodiment without syringe showing front and side views;
Figure 12 illustrates a syringe finger rest attach embodiment, without syringe;
Figure 13 illustrates a syringe with and without syringe finger rest attach embodiment, with - side view;
Figure 14 illustrates a syringe with and without syringe finger rest attach embodiment, with - front view;
Figure 15 illustrates a syringe puck embodiment, with syringe;
Figure 16 illustrates a syringe puck embodiment, without syringe
Figure 17 illustrates a trainer evoke embodiment;
Figure 18 illustrates a packaging attachment embodiment A, attached to syringe while in tray;
Figure 19 illustrates a packaging attachment embodiment A, detached from syringe;
Figure 20 illustrates a packaging attachment embodiment B, attached to syringe tray;
Figure 21 illustrates a packaging attachment embodiment B, detached from syringe tray;
Figure 22 is a chart illustrating the threshold requirement for a device containing sediment;
Figure 23 is a chart illustrating how a proportion of users will fail to provide sufficient vigor to adequately mix the product un-aided;
Figure 24 is a chart illustrating the role of a device or aid, to modify user behavior so all shake with sufficient vigor to exceed the require threshold;
Figure 25 illustrates a syringe case embodiment with LCD display - sedimentation mixing represented using display;
Figure 26 illustrates LCD screen conditions showing stages of mix from sediment on left to mix on the right;

The accompanying drawings, which are incorporated herein and constitute part of this specification, illustrate presently preferred embodiments of the invention, and, together with the general description given above and the detailed description given below, serve to explain features of the invention (wherein like numerals represent like elements).

### Modes of Carrying Out the Invention

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

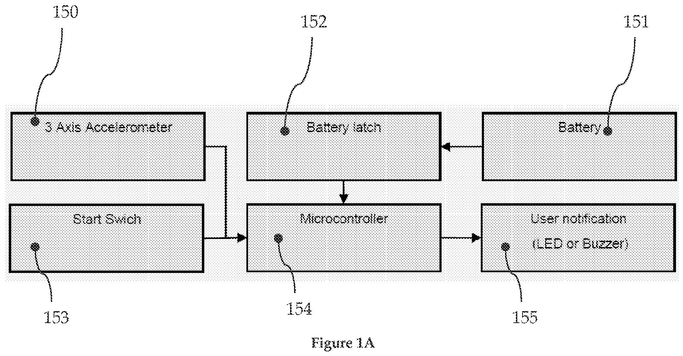

The exemplary embodiments shown and described here may utilize an electronic system such as an electronic circuit mounted on a printed circuit board (PCB), which may include means to supply and control electrical power, means to measure spatial acceleration, means to time the duration of shaking and means to communicate the device state to the user as shown in Figure 1A.

Figure 1A illustrates a schematic overview of the electronic system that can be utilized with various embodiments described and illustrated herein. It is noted that for these embodiments, the forces are measured preferably using a 3-axis accelerometer 150 because the user's shaking movement will likely be in more than one direction and furthermore sufficient acceleration may be a factor of accelerations in multiple directions. Hence, a threshold may be determined to be a function of one or more acceleration directions. Additionally the system may include a start switch 153, which may be a manually activated switch, or preferably may be an acceleration activated switch to wake up the circuit, thus conserving battery power when the device is not in use. The advantage of the acceleration activated switch is that the user need only start shaking the device to switch it on. In some embodiments it may be preferable to require the user to make a conscious decision to switch the device on, and carry out a specific explicit action to switch the device on, hence the manual switch may be preferable. Once the device has woken up, the battery latch 152 ensures power is supplied from the battery 151 for sufficient time to complete the shake cycle and provide feedback to the user.

A further development of this embodiment is described as follows. Time is measured using a microcontroller 154, microprocessor or timer; from the moment that shaking is first detected. The electronic system is programmed with an algorithm which compares the measured shake acceleration and duration with a preset threshold. The electronic system compares the recorded elapsed time and force measurements and retums a communication signal corresponding to the device state 155. The device state is communicated using one or preferably a combination of the following: visual feedback such as light emitting diode (LED), graphical display such as Liquid Crystal Display (LCD); audible feedback such as an audible buzzer or polyphonic speaker; tactile feedback such as vibration motor. Such an electronic circuit requires a power source, such as a battery, that may be rechargeable or not. If not rechargeable, the battery may be replaceable or it may not be replaceable, in which case the device must be disposed of in its entirety at end of life, defined by the end of the battery life.

The electronic circuit is contained inside the device, with visual feedback means visible to the user by way of a window or light transmitting element. The electronic circuit affords additional design features:

**Pause function.** The program allows the system to detect when shaking has ended prematurely, or if the level of shaking vigor has reduced to a level below the pre-set threshold, using the accelerometer or acceleration activated switch. If such events are detected the device may enter a pause mode, which pauses the timing process and may indicate to the user that the device has entered pause mode. The indication to the user may be a pause in the feedback being provided, such as a pause in the audible tone, or a pause in the tactile vibration; or it may be by other means such as a state change on a light or screen. The user, upon receiving the pause indication may then correct their actions, by recommencing the shake action or increasing the vigor of the shake action, at which moment the device switches out of pause mode and recommences with the timing and force monitoring process, starting from the time count at which it paused. If the device is in pause mode for a significant amount of time such that the particles may have started to re-form a sediment, the timer will be reset.

**Syringe administration timer.** A timer may be used to warn the user that too much time has elapsed since the device was shaken. After shaking, the particles will slowly return to a sediment state; therefore there is a maximum time limit between shaking the device and administering the injection. In some scenarios, the user may shake the device correctly, but then may be distracted long enough for the particles in the syringe to re- sediment. A timer may be set such that when the max allowable time after shaking of the housing has elapsed, the user is warned to shake the device again before administration. This warning could be communicated using several methods. As examples, an audible buzzer could sound when the maximum time has elapsed, or a green light which was illuminated to indicate shaking has completed could switch off.

**Low battery warning.** Such an electronic system requires a power source, preferably a battery cell. When the remaining power in the battery has reduced to a point where device functionality may soon become impaired, the device may indicate to the user that a battery failure is imminent, and that the battery must be replaced. The device could shut down in such an event so that it may not be used until the battery has been replaced. This would prevent a potential device malfunction.

**Error warning.** Such an electronic system can perform self-checks on the system and main components so that when errors are detected the user may be given warning. It can perform such self-checks whenever the device is awoken for a shake cycle and immediately communicate that it has entered an error mode. This prevents the user from using a faulty device and prompts them to take remedial action, for example to return the device to the manufacturer, and use a replacement device.

**Information distinguishing force and time.** In the first instance this invention is described as providing the user with information on the success of the shake action as a single piece of information, when both sufficient time AND sufficient vigor have been achieved. An alternative arrangement of such an electronics system can provide feedback to the user on the constituent elements; in that two pieces of information are provided, the elapsed time and the level of vigor over that time. That way if a user fails to achieve the combination of sufficient time and vigor they may consult the information provided and determine the reason they were unable to be successful, they may determine if they failed to shake for sufficient time, or if they failed to shake with sufficient vigor.

**Drug expiration alert.** A development of such an electronic system incorporates means to read the expiration data on the syringe and warn the user if it has expired. If the user were informed that the expiration date had expired they could dispose of the expired syringe and use another that had not expired. Such a system uses scanning components that read a barcode or text on the syringe to gather the expiration date, or may communicate with a chip on the syringe that contains the lot and expiration information, and compares the date to an internal clock and calendar programmed into the processor and gives an alert if the gathered date is before that on the internal clock and calendar.

The features described and illustrated above can be embodied in the following Embodiments 1-8 with highlights in the variations and differences between each embodiment described with reference to the drawing figures indicated below.

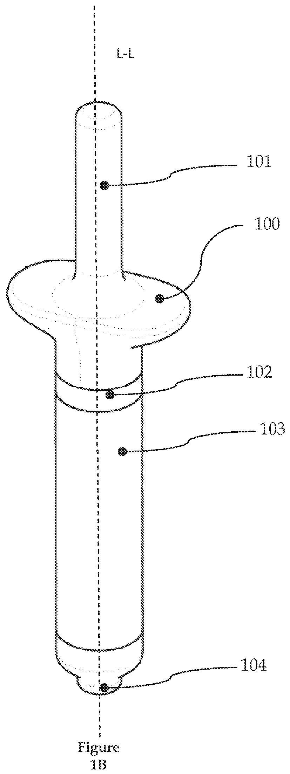

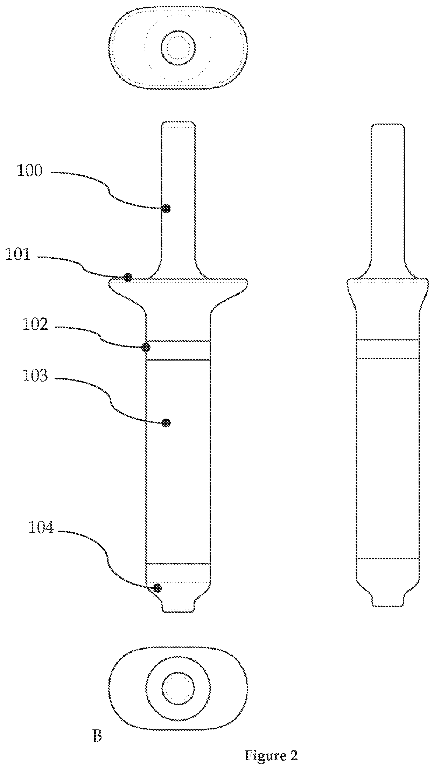

**Embodiment 1. MIMIC.** The trainer embodiment (Figure 1B and Figure 2) uses a form factor for a housing that mimics that of a syringe. It may include a syringe barrel element 103, with finger flange 100 at one end and a barrel tip 104 at the other. The distance between finger flange 100 and barrel tip 104 are similar to that of a syringe such that it may be held in a similar fashion. At the top of the form is a cylindrical portion 100 that represents a syringe plunger rod. The barrel 103 includes a light emitting feedback window 102, through which light is emitted to communicate the state of the device. In the preferred embodiment, amber light is used to indicate the device is running through a shake cycle and is monitoring the level of agitation imparted on it; green light is used to indicate the shake cycle is complete and sufficient shaking has occurred. When not in use, the light is off to conserve battery power and also to indicate that the shake cycle has not yet commenced.

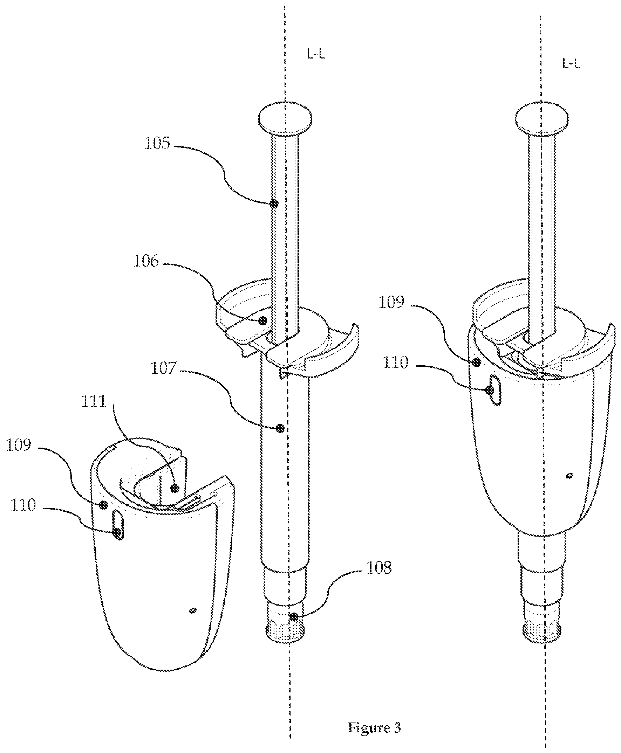

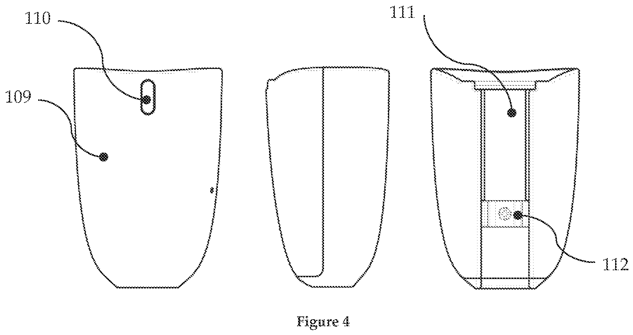

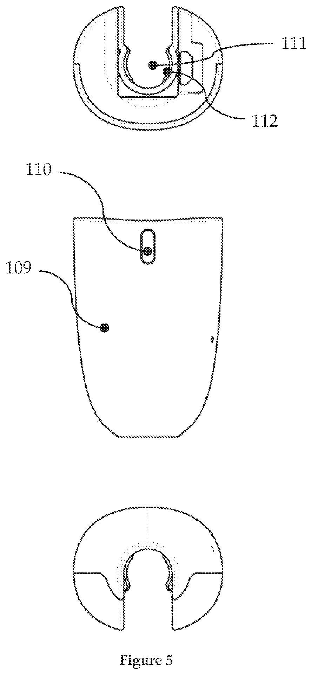

**Embodiment 2** - **ATTACH.** The embodiment shown in Figure 3 - Figure 5 attaches to the syringe and provides feedback to the user while they are shaking the syringe. The device housing may include a body 109 with a slot 111 sized to accept a syringe barrel 107. A clip 112 (Figure 4) in the slot 111 retains the syringe. Once the device housing is attached to a syringe, the shaking motion is measured using the internal electronic system, and the device state is communicated in two simultaneous ways. The first communication method is with light emitted from the light emitting feedback window 110. An amber light indicates that a shake cycle is in progress, and a green light indicates that it is completed. The second simultaneous feedback method is audible. An intermittent audible buzzing tone indicates that the shake cycle is in progress, which changes to a continuous tone when the shake cycle is completed. Furthermore, the intermittent tone emitted during the shake cycle is set at a frequency of approximately 3Hz to reinforce the frequency and speed of shake required for optimum mixing, exploiting the tendency for humans to match repetitive behavior to percussive audible tones.

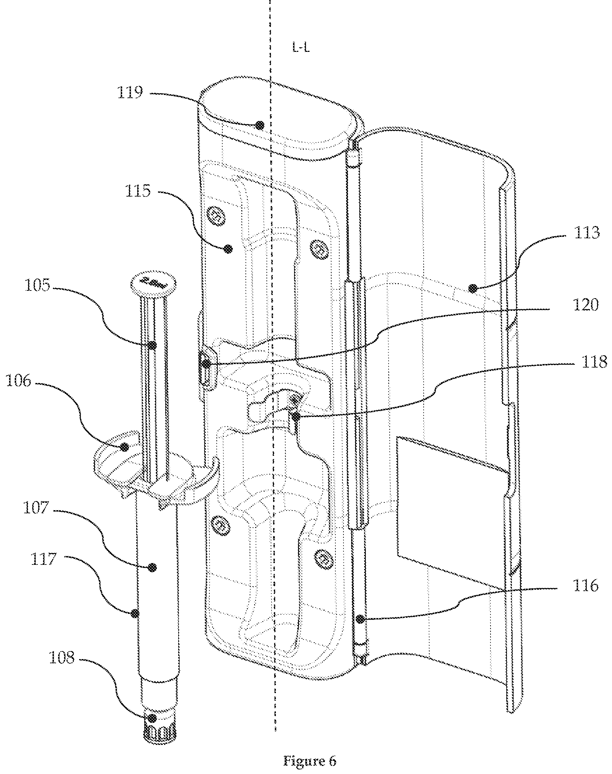

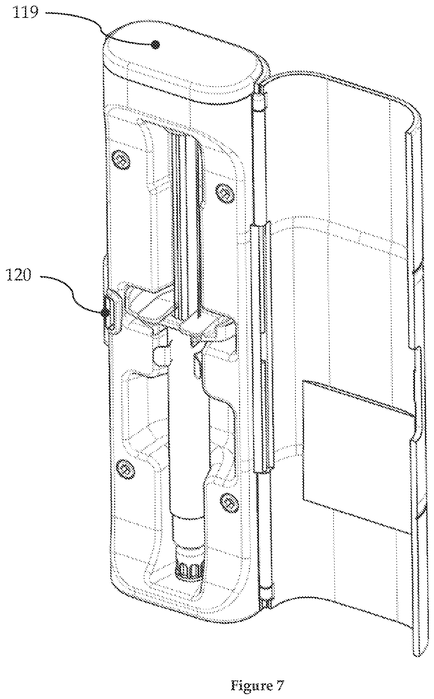

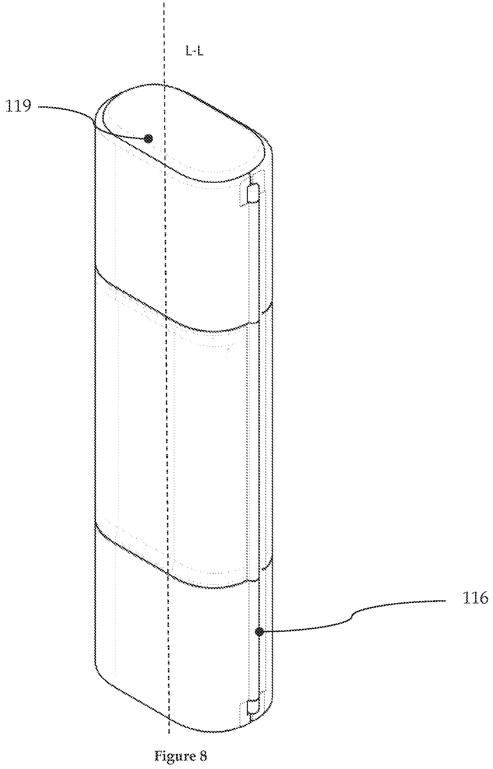

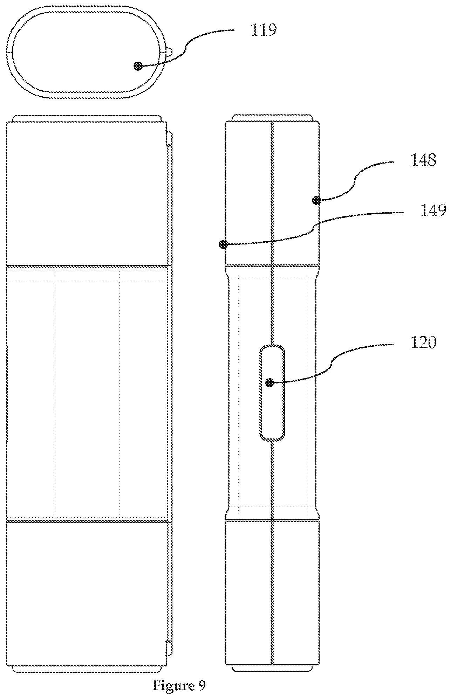

**Embodiment 3 - CASE.** The embodiment shown in Figure 6 - Figure 9 contains the syringe and provides feedback to the user while they are shaking the syringe. The device housing is opened by pressing on the lid catch 120; a syringe 117 is placed in the case liner 115 where it is retained by a spring clip 118 as shown in Figure 7. Alternatively, the syringe could be retained by case lid 113 when the device housing is closed. Case lid 113 pivots around the case syringe 116 enclosing the syringe as shown in Figure 8. The case includes flat surfaces on the case base 148 and case top 149 (Figure 9), such that when placed on a desktop or work top it will not roll off. The case is closed and retained in a closed position by the lid catch 120. The outer shape of the case is designed to allow for users to hold it comfortably and securely when shaking. Shaking motion is measured using the internal electronic system, and the device state is communicated in two simultaneous ways. The first communication method is with light emitted from the light emitting panel 119, and amber light indicates that a shake cycle is in progress; and a green light indicates that it is completed. The second simultaneous feedback method is tactile. An intermittent vibration transmitted to the holding hand indicates that the shake cycle is in progress which changes to a continuous vibration when the shake cycle is completed. Furthermore the intermittent vibration emitted during the shake cycle is set at a frequency of approximately 3Hz to reinforce the frequency and speed of shake required for optimum mixing, exploiting the tendency for humans to match repetitive behavior to percussive stimuli. The light emitting panel turning green indicates to the user to remove the syringe and continue with the drug administration process. This embodiment may be further developed with a syringe lock out feature. This feature would detect the presence of the syringe inside, lock the case in the closed state and only unlock the case when sufficient time and shake vigor have been achieved. In addition, if the user does not open the case to remove the syringe for injection within a specified time period, the case could re-lock itself, and/or the light could turn amber again, indicating that the device housing must be shaken again to re-suspend the particles before the syringe can be used.

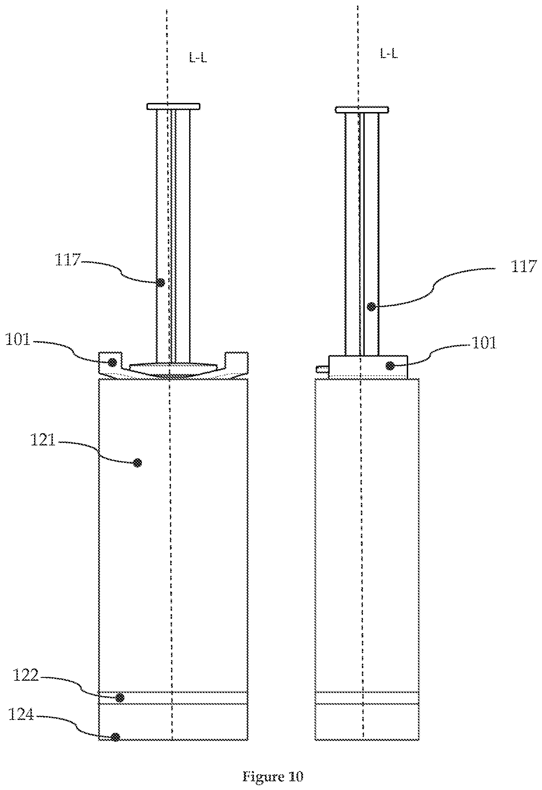

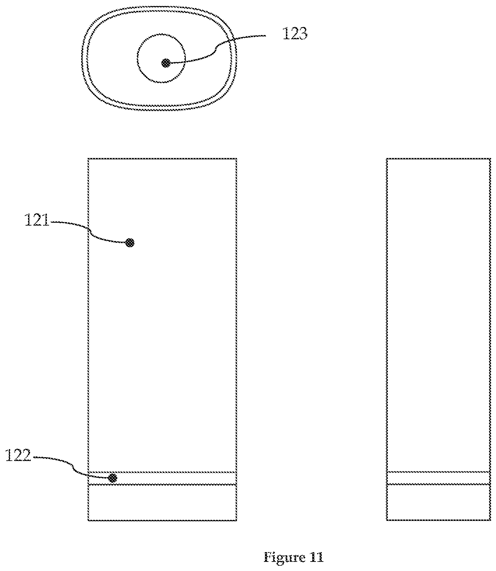

**Embodiment 4** - **POT.** The embodiment shown in Figure 10 and Figure 11 includes an elongated body housing approximately the same length as the syringe barrel. The syringe is inserted into a syringe receiving hole 123 in the body housing and is retained by the compressive force applied by the fingers between the syringe finger flange 101 and the device body housing base 124. When the coupled device and syringe are shaken, motion is measured using the internal electronic system, and the device state is communicated in two simultaneous ways. The first communication method is with light emitted from the light emitting band 122, and amber light indicates that a shake cycle is in progress; and a green light indicates that it is completed. The second simultaneous feedback method is tactile. An intermittent vibration transmitted to the holding hand indicates that the shake cycle is in progress which changes to a continuous tone when the shake cycle is completed. Furthermore, the intermittent vibration emitted during the shake cycle is set at a frequency of approximately 3Hz to reinforce the frequency and speed of shake required for optimum mixing, exploiting the tendency for humans to match repetitive behavior to percussive stimuli. The light emitting panel turning green indicates to the user to remove the syringe and continue with the drug administration process. As with other embodiments, further means of supplemental communication may be included, such as audible or tactile vibrating feedback, and a timing function can be used to signal the user if the syringe has not been removed from the device before a specified time period, indicating that the device must be shaken again to re-suspend the particles prior to performing the injection. A switch internal to receiving hold 123 would be used to detect when the syringe is attached to the device.

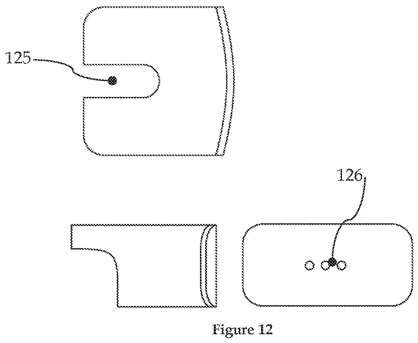

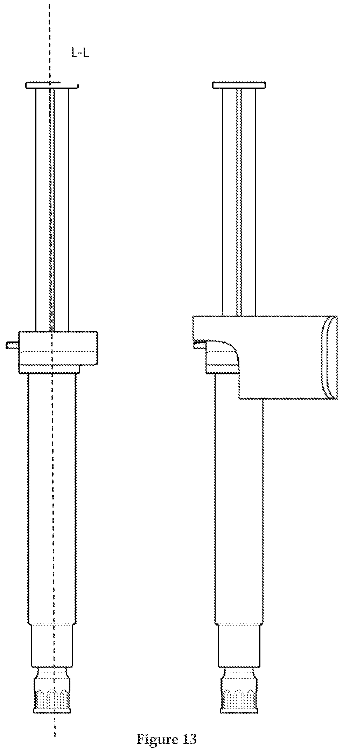

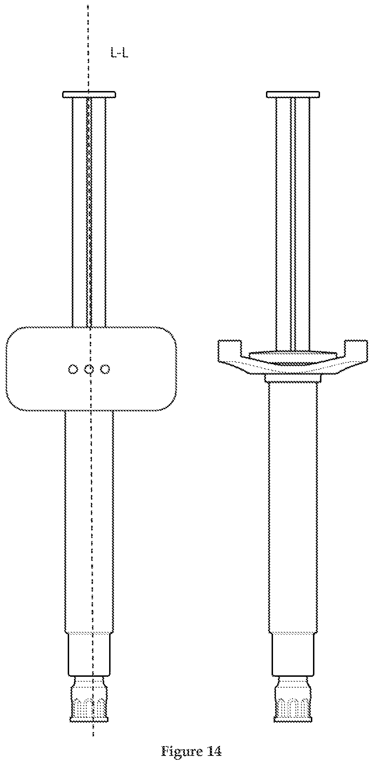

**Embodiment 5 - FINGER REST ATTACHMENT.** The embodiment shown in Figure 12, Figure 13 and Figure 14 is similar to embodiment 2, in that it attaches to the syringe. It is shown on the syringe in Figure 13 and Figure 14. The syringe fits and is retained in the syringe accepting slot 125. The electronic system is housed within, and communicates to the user via the light panel 126 on the front face. An orange color indicates when the device is sensing and this turns green when it has been shaken sufficiently. As with other embodiments, further means of supplemental communication may be included, such as audible or tactile vibrating feedback.

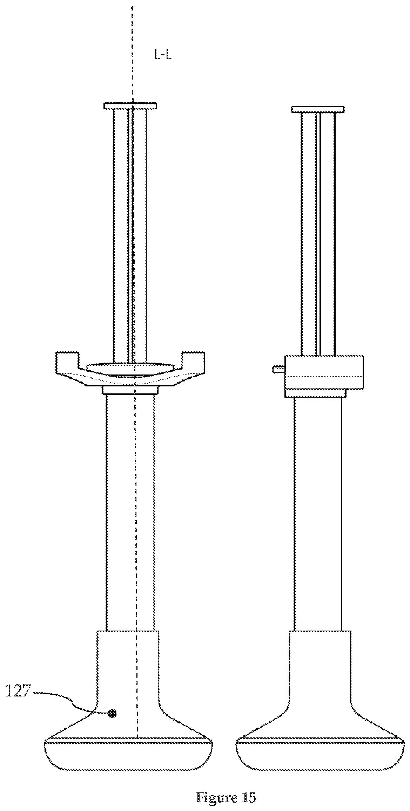

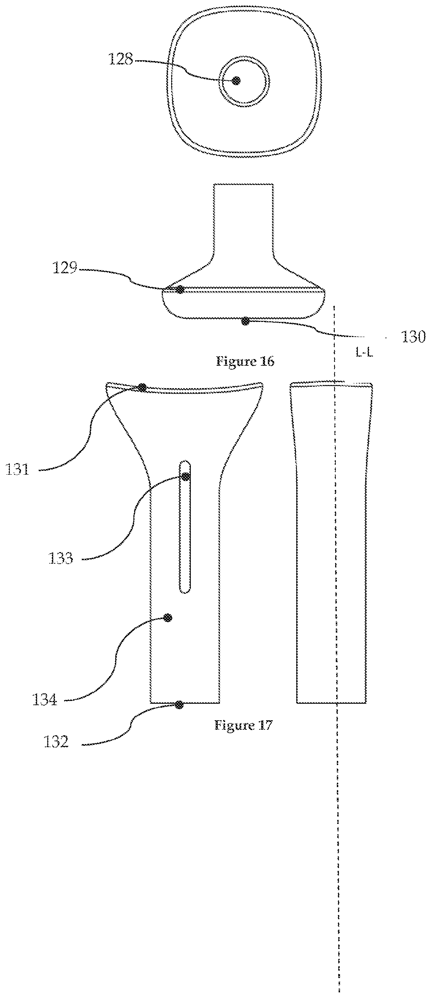

**Embodiment 6 - PUCK.** The embodiment shown in Figure 15 and Figure 16 is similar to embodiment 4, in that it fits to the bottom of the syringe but is a much more compact design. It may include a body housing 127 with syringe accepting hole 128. The interface is provided by a circumferential light emitting band 129 around the diameter of the base of the form. In use, the syringe is inserted into the syringe accepting hole 128 and is held in place with the user's thumb on the underside thumb grip 130. As with other embodiments, further means of supplemental communication may be included, such as audible or tactile vibrating feedback.

**Embodiment 7 - EVOKE.** The embodiment shown in Figure 17 is similar to embodiment 1 of Figure 1B and 2. It is a stand-alone device, which does not interact with the syringe, intended to be used in advance of the administration process. The user may practice shaking in advance of shaking the real syringe. It may include the device body housing 134, at either end of is the finger grip 131 and the thumb grip 132. The distance between finger grip and thumb grip is similar to the distance between the syringe finger grip and rubber stopper, to ensure it feels similar in the hand. When the user shakes the device, the device detects the forces applied and emits an amber light from the light emitting window 133. The light turns green when sufficiently vigorous shaking has occurred for sufficient time. As with other embodiments, further means of supplemental communication may be included, such as audible or tactile vibrating feedback.

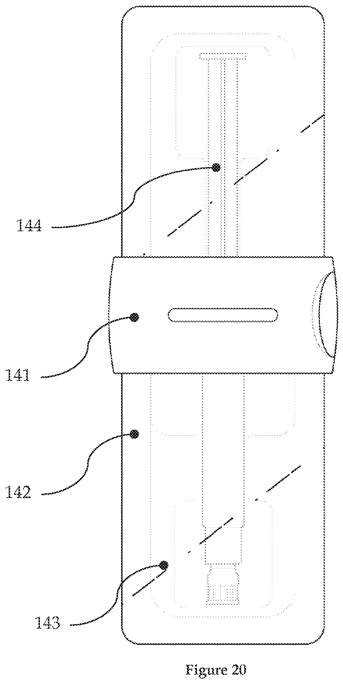

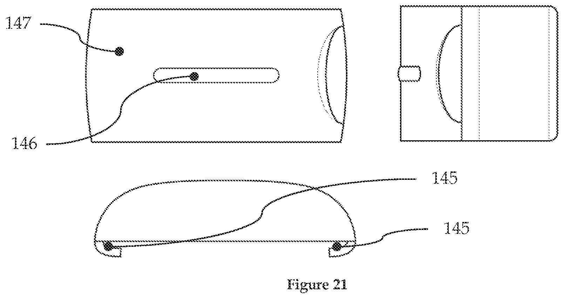

**Embodiment 8 - PACKAGING B.** With reference to Figure 20 and Figure 21, this embodiment is similar to embodiment 8, in that the device 141 attaches to the syringe blister packaging 142 while the syringe 144 is contained within. In this embodiment, the protective film 143 is still in place on top of the blister tray 142. With reference to Figure 21 the device is attached to the blister tray by sliding it along the length of the blister tray and is retained against the blister tray by means of return clips 145 on the underside of the device. The body housing of the device 147 includes means of communication, a light emitting strip 146. As with other embodiments the electronic system in the device detects the onset of shaking and starts to monitor the vigor and duration of shaking. While doing so, the light emitting strip 146 emits amber light. Once sufficient vigor and duration have been achieved the light changes to green. After shaking is completed the device is removed from the blister tray 142; the blister tray film 143 is peeled off and the device is used normally as per the syringe administration instructions. As with other embodiments, further means of supplemental communication may be included, such as audible or tactile vibrating feedback.

It is noted that during formulation development for Invega Sustenna Three Month, the required duration and vigor required were identified and quantified as 15 seconds of vigorous shaking. Vigorous shaking was initially defined using a training video in which an expert experienced in the correct preparation of the pharmaceutical product shakes a syringe for the required 15 seconds at the required level of vigor. Video analysis was used to estimate the amplitude and frequency of the demonstrated shake, finding that shake amplitude of approximately 40cm was used at a frequency of 3.4 hertz. Assuming simple harmonic motion it was calculated that the syringe was experiencing a maximum acceleration of 9.3g. This provides an indication of the required accelerations imparted on the fluid to achieve sufficient mixing as recommended by an expert in the preparation of such a formulation.

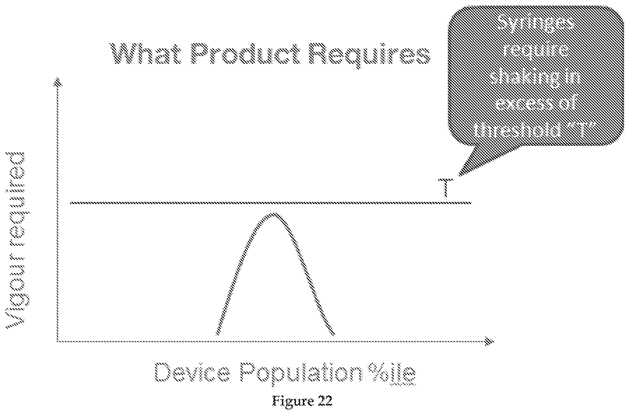

The failure mode associated with not mixing the syringe sufficiently is a failure to administer the full dose from the syringe. This occurs because insufficiently shaken syringes contain residual sediment which can block, or partially block, the syringe or needle during administration. Therefore syringes containing fluids with different sediment properties such as mass, density, and concentration can be quantified in terms of the required mixing vigor by applying controlled and known levels of acceleration (by experimental means) measuring the force required to eject the fluid from a syringe. Using such methods, different shake vigor thresholds may be determined for different fluid with different properties. Figure 22 illustrates the device requirement for a threshold exceeding the intrinsic variability in the device population.

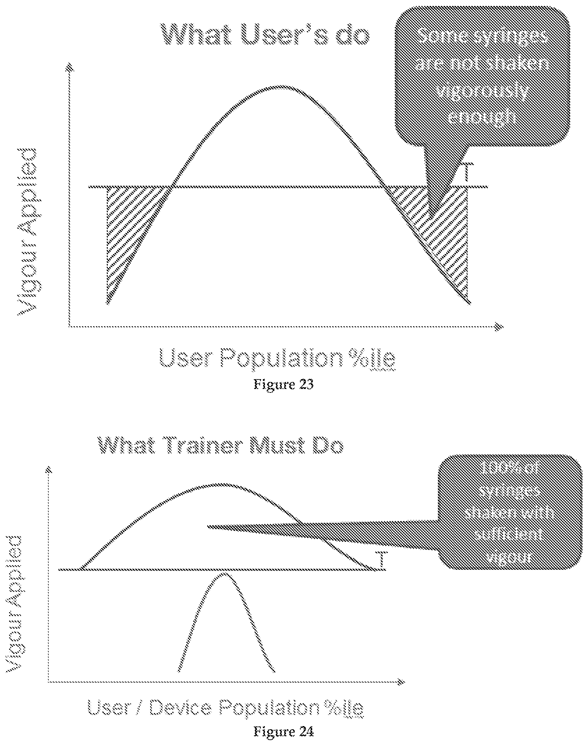

Once the required threshold is determined, the behavior of users should also be understood. Different users will have different capabilities, strengths, habits and expectations; therefore, there is in inherent variability in the way each interprets the instruction "shake vigorously" and some will naturally fail to meet the required threshold as illustrated in the chart in Figure 23. Moreover, different users employ different shaking techniques, which some of which will be more vigorous than others. Acknowledging this variability, and that a minimum shake time and vigor are required leads to the conclusion that a need exists for the device described in this disclosure. The device can communicate the required time and vigor to the user, and modify behavior, increasing the likelihood of the user achieving the minimum required level of mixing. The subsequent effect of the device on the behavior across a population of users is illustrated in Figure 24.

Once the threshold requirements and behavior modification goals are understood, consideration can be given to the various embodiments of form and function that might elicit the desired behavior modification. Such embodiments may either be used as a stand-alone device or as an in process device.

A stand-alone device is used in isolation of the administration process, providing the user an opportunity to shake a device and learn what level of vigor is required when they come to shake the real device. Such a stand-alone device may have a form factor close to that of the syringe so it may represent the experience of shaking the real syringe a closely as possible. Other form factors may also be used. For example, if the drug comes in a vial, a vial shaped device would be more appropriate.

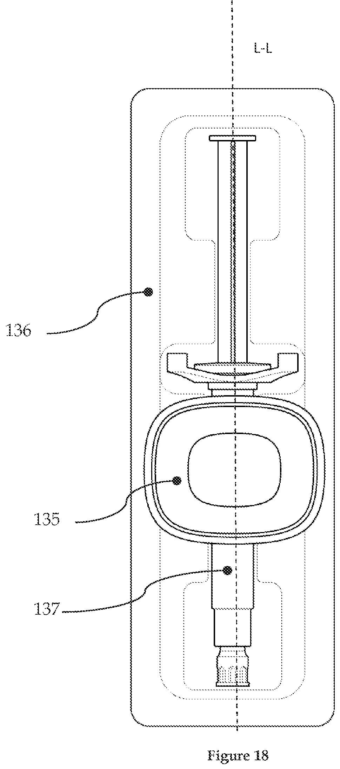

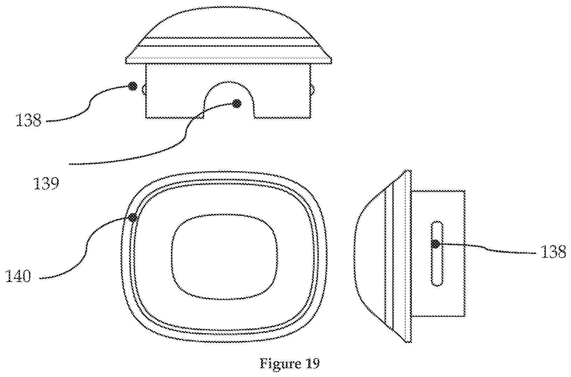

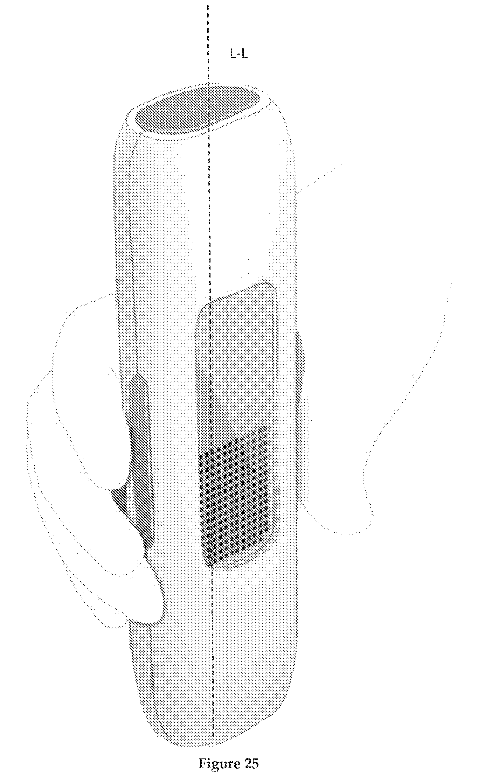

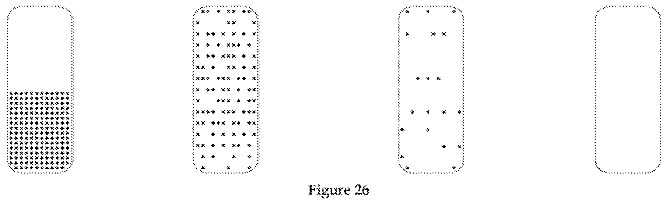

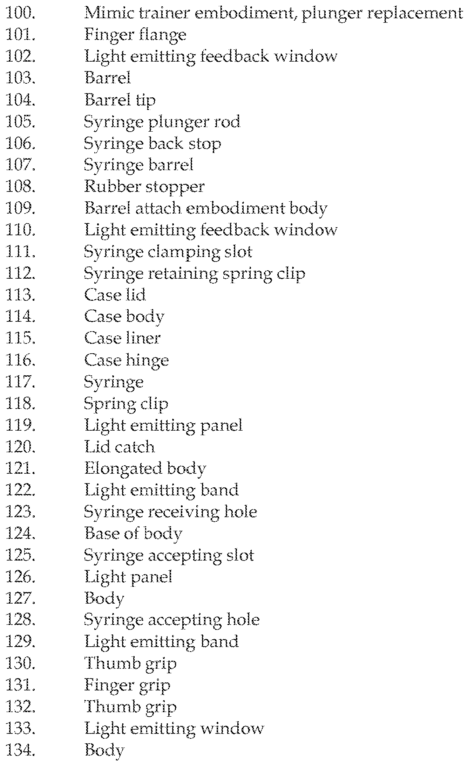

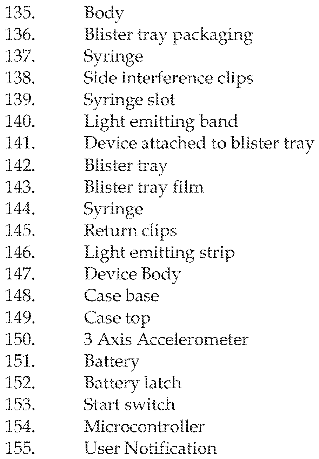

An assistive device is required to couple with, attach to, or encase the real syringe. As such means of coupling, attaching or encasing are required. This leads to varied opportunities for several form factors illustrated in figures 1-21. Several means of communicating the device state to the user are possible. As previously described, they may include lights of different color or state (flashing or steady state); they may include audible means such as buzzers and speakers; and they may include tactile means such as vibrations. Further means are possible as shown in Figure 25, such as a LCD (liquid Crystal Display) or similar, which may use graphical means to communicate the device state. An LCD may be used as a segment display to communicate device state through words and icons, or it may be used as a metaphorical indication of the mix state of the solution such as described in Figure 26. In this case, LCD segments or pixels are switched on or off to create a visual indication of particles mixing with solution. On the left side of Figure 26, darkly colored pixels in the lower portion of the display represent the presence of sediment in the bottom of the syringe. When shaking is detected by the electronic system, different pixels are switched on and off across the whole screen to indicate mixing is in progress, and when sufficient mixing is achieved the LCD can show a uniform homogenous color across the whole screen, indicating that the solution is also in a uniform homogeneous state.

By virtue of various embodiments of the invention, certain benefits were realized where the invention is configured as a stand-alone training device: (a) It makes the user aware of what the required shake time is; they learn through experience, (b) It allows the user to experience what the required level of shake vigor is, (c) It teaches the user what duration and level of vigor is required for the real device without impeding the normal administration process flow (d), It allows a device to have a form factor very close to that of the real syringe, (c), It allows the user to develop their skills so they are able to shake syringe sufficiently without having to rely on assistive aids, and (f) It is independent of the actual injection process and thus does not overly complicate it.

Other benefits were also realized when the invention is configured as an actual or "in-process" device: (a) It makes the user aware of what the required shake time is; they learn through experience, (b) It allows the user to experience what the required level of shake vigor is, (c) When attached to the syringe it allows the actual syringe to be shaken, providing the user with real time reassurance that it has been shaken adequately, and (d) If used correctly it reduces the chance of a real syringe being shaken insufficiently.

It is noted that these embodiments have been prototyped for testing with multiphase injectable pharmaceutical solutions. From such testing, a version has been selected for commercialization with a product with the trademark of INVEGA TRINZA^{™}, which product is planned for distribution by Janssen Pharmaceuticals, Inc. Titusville, NJ 08560. It is also noted that the proposed commercial version of the invention is intended to be utilized with INVEGA TRINZA^{™} in which a copy of the product label is attached to the appendix of this application, and such product label for INVEGA TRINZA^{™} is intended to be part of this patent application.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above.

### Appendix

### HIGHLIGHTS OF PRESCRIBING INFORMATION

These highlights do not include all the information needed to use INVEGA TRINZA^{™} safely and effectively. See full prescribing information for INVEGA TRINZA^{™}.

INVEGA TRINZA^{™} (paliperidone palmitate) extended-release injectable suspension, for intramuscular use Initial U.S. Approval: 2006

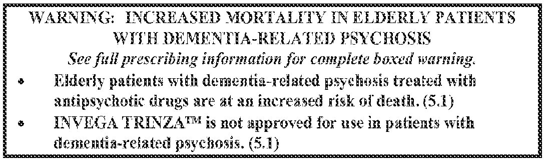

### INDICATIONS AND USAGE

INVEGA TRINZA^{™}, a 3-month injection, is an atypical antipsychotic indicated for the treatment of schizophrenia in patients after they have been adequately treated with INVEGA SUSTENNA^{®} (1-month paliperidone palmitate extended-release injectable suspension) for at least four months. (1)

### DOSAGE AND ADMINISTRATION

- Use INVEGA TRINZA^{™} only after the patient has been adequately treated with the 1-month paliperidone palmitate extended-release injectable suspension for at least four months. (2.2)
- INVEGA TRINZA^{™} should be administered once every 3 months. (2.1)
- For intramuscular injection only. (2.1)
- Each injection must be administered only by a health care professional. (2.1)
- For deltoid injection: For patients weighing less than 90 kg, use the 1-inch 22 gauge thin wall needle. For patients weighing 90 kg or more, use the 1½-inch 22 gauge thin wall needle.
- For gluteal injection: Regardless of patient weight. use the1½-inch 22 gauge thin wall needle.
- Prior to administration. shake the prefilled syringe vigorously for at least 15 seconds within 5 minutes prior to administration to ensure a homogeneous suspension. (2.1)
- Initiate INVEGA TRINZA^{™} when the next 1-month paliperidone palmitate dose is scheduled with an INVEGA TRINZA^{™} dose based on the previous 1-month injection dose as shown below. (2.2)

### INVEGA TRINZA^{™} Doses for Adult Patients Adequately Treated with INVEGA SUSTENNA^{®}

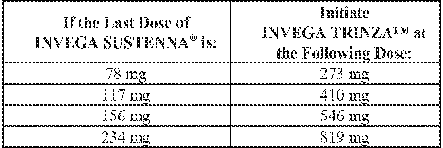

Conversion from the INVEGA SUSTENNA^{®} 39 mg dose was not studied.
- Missed Doses: Missing doses of INVEGA TRINZA^{™} should be avoided. To manage missed doses on exceptional occasions, refer to the Full Prescribing Information. (2.3)
- Moderate to severe renal impairment (creatinine clearance < 50 mL min): INVEGA TRINZA^{™} is not recommended. (2.5)
- Mild renal impairment (creatinine clearance ≥ 50 mL/min to < 80 mL/min): Adjust dosage and stabilize the patient using INVEGA SUSTENNA^{®} then transition to INVEGA TRINZA^{™}. See above table. (2.5)

### DOSAGE FORMS AND STRENGTHS

Extended-release injectable suspension: 273 mg, 4 10 mg, 546 mg, or 819 mg (3)

### CONTRAINDICATIONS

Known hypersensitivity to paliperidone, risperidone, or to any excipients in the formulation (4)

### WARNINGS AND PRECAUTIONS

- *Cerebrovascular Adverse Reactions*, *Including Stroke*, *in Elderly Patients with Dementia- Related Psychosis:* Increased incidence of cerebrovascular adverse reactions (e.g. stroke, transient ischemic attack, including fatalities). INVEGA TRINZA^{™} is not approved for use in patients with dementia-related psychosis (5.2)
- *Neuroleptic Malignant Syndrome:* Manage with immediate discontinuation of drug and close monitoring (5.3)
- *QT Prolongation:* Avoid use with drugs that also increase QT interval and in patients with risk factors for prolonged QT interval (5.4)
- *Tardive Dyskinesia:* Discontinue drug if clinically appropriate (5 5)
- *Metabolic Changes:* Atypical antipsychotic drugs have been associated with metabolic changes that may increase cardiovascular/cerebrovascular risk. These metabolic changes include:
   ∘ *Hyperglycemia and Diabetes Mellitus:* Monitor for symptoms of hyperglycemia including polydipsia, polyuria, polyphagia, and weakness. Monitor glucose regularly in patients with diabetes or at risk for diabetes. (5.6)
   ∘ *Dyslipidemia:* Undesirable alterations have been observed. (5.6)
   ∘ *Weight Gain:* Significant weight gain has been reported. Monitor weight gain. (5.6)
- *Orthostatic Hypotension and Syncope:* Use with caution in patients with known cardiovascular or cerebrovascular disease and patients predisposed to hypotension (5.7)
- *Leukopenia*, *Neutropenia*, *and Agranulocytosis:* Monitor complete blood count in patients with a history of a clinically signiticant low white blood cell count (WBC) or a drug-induced leukopenia/neutropenia. Consider discontinuation if clinically significant decline in WBC in the absence of other causative factors (5.8)
- *Hyperprolactinemia:* Prolactin elevations occur and persist during chronic administration (59)
- *Potential for Cognitive and Motor Impairment:* Use caution when operating machinery (5.10)
- *Seizures:* Use cautiously in patients with a history of seizures or with conditions that lower the seizure threshold (5.11)

### ADVERSE REACTIONS

The most common adverse reactions (incidence ≥ 5% and occusting at least twice as often as placebo) were injection site reaction, weight increased, headache, upper respiratory tract infection, aknthisia, and parkinsonism. (6)

### To report SUSPECTED ADVERSE REACTIONS, contact Janssen Pharmaceuticals, Inc. at 1-800-JANSSEN (1-800-526-7736) or FDA at 1-800-FDA-1988 or www.fda.gov/medwatch

### DRUG INTERACTIONS

*Strong CYP3A4*/*P-glycoprotein (P-gp) inducers:* Avoid using a strong inducer of CYP3A4 and/or P-gp (e.g., carbamazepine, rifampin, St John's Wort) during a dosing interval for INVEGA TRINZA^{™}. If administering a strong inducer is necessary, consider managing the patient using paliperidone extended release tablets. (7.2, 12.3)

### USE IN SPECIFIC POPULATIONS

*Pregnancy:* May cause extrapyramidal and/or withdrawal symptoms in neonates with third trimester exposure. (8.1)

See 17 for PATIENT COUNSELING INFORMATION and FDA-approved patient labeling.

### FULL PRESCRIBING INFORMATION: CONTENTS

### WARNING: INCREASED MORTALITY IN ELDERLY PATIENTS WITH DEMENTIA-RELATED PSYCHOSIS

**1 INDICATIONS AND USAGE**
**2 DOSAGE AND ADMINISTRATION**
   2.1 Administration Instructions
   2.2 Schizophrenia
   2.3 Missed Doses
   2.4 Use with Risperidone or with Oral Paliperidone
   2.5 Dosage Adjustment in Renal Impairment
   2.6 Switching from INVEGA TRINZA^{™} to the 1-Month Palipendone Palmitate Extended-Release Injectable Suspension
   2.7 Switching from INVEGA TRINZA^{™} to Oral Paliperidone Extended-Release Tablets
   2.8 Instructions for Use
**3 DOSAGE FORMS AND STRENGTHS**
**4 CONTRAINDICATIONS**
**5 WARNINGS AND PRECAUTIONS**
   5.1 Increased Mortality in Elderly Patients with Dementia-Related Psychosis
   5.2 Cerebrovascular Adverse Reactions, Including Stroke, in Elderly Patients with Dementia-Related Psychosis
   5.3 Neuroleptic Malignant Syndrome
   5.4 QT Prolongation
   5.5 Tardive Dyskinesia
   56 Metabolic Changes
   5.7 Orthostatic Hypotension and Syncope
   5.8 Leukopenia, Neutropenia, and Agranulocytosis
   5.9 Hyperprolactinemia
   5.10 Potential for Cognitive and Motor Impairment
   5.11 Seizures
   5.12 Dysphagia
   5.13 Priapism
   5.14 Disruption of Body Temperature Regulation
**6 ADVERSE REACTIONS**
   6.1 Clinical Trials Experience
   6.2 Postmarketing Experience
**7 DRUG INTERACTIONS**
   7.1 Drugs Having Clinically Important Interactions with INVEGA TRINZA^{™}
   7.2 Drugs Having No Clinically Important Interactions with INVEGA TRINZA^{™}
**8 USE IN SPECIFIC POPULATIONS**
   8.1 Pregnancy
   8.2 Lactation
   8.4 Pediatric Use
   8.5 Geriatric Use
   8.6 Renal Impairment
   8.7 Hepatic Impairment
   8.8 Patients with Parkinson's Disease or Lewy Body Dementia
**9 DRUG ABUSE AND DEPENDENCE**
   9.1 Controlled Substance
   9.2 Abuse
   93 Dependence
**10 OVERDOSAGE**
   10.1 Human Experience
   10.2 Management of Overdosage
**11 DESCRIPTION**
**12 CLINICAL PHARMACOLOGY**
   12.1 Mechanism of Action
   122 Pharmacodynamics
   12.3 Pharmacokinetics
**13 NONCLINICAL TOXICOLOGY**
   13.1 Carcinogenesis, Mutagenesis. Impairment of Fertility
   13.2 Animal Toxicology and/or Pharmacology
**14 CLINICAL STUDIES**
**16 HOW SUPPLIED/STORAGE AND HANDLING**
**17 PATIENT COUNSELING INFORMATION**

*Sections or subsections omitted from the full prescribing information are not listed

### FULL PRESCRIBING INFORMATION

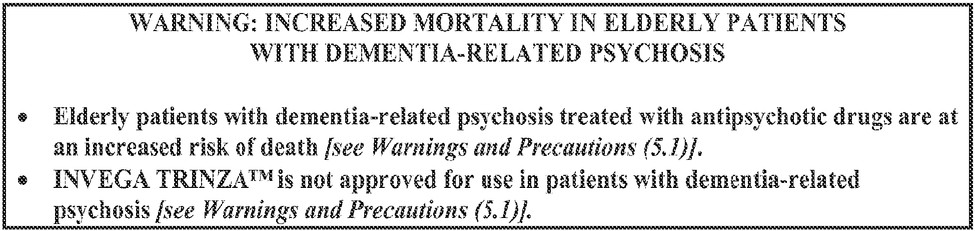

### 1 INDICATIONS AND USAGE

INVEGA TRINZA^{™} (paliperidone palmitate), a 3-month injection, is indicated for the treatment of schizophrenia in patients after they have been adequately treated with INVEGA SUSTENNA^{®} (1-month paliperidone palmitate extended-release injectable suspension) for at least four months *[see Dosage and Administration (2.2) and Clinical Studies (14)].*

### 2 DOSAGE AND ADMINISTRATION

### 2.1 Administration Instructions

INVEGA TRINZA^{™} should be administered once every 3 months.

Each injection must be administered only by a health care professional.

Parenteral drug products should be inspected visually for foreign matter and discoloration prior to administration. **It is important to shake the syringe vigorously for at least 15 seconds to ensure a homogeneous suspension. Inject INVEGA TRINZA^{™} within 5 minutes of shaking vigorously** *[see Dosage and Administration (2.8)].*

INVEGA TRINZA^{™} is intended for intramuscular use only. Do not administer by any other route. Avoid inadvertent injection into a blood vessel. Administer the dose in a single injection; do not administer the dose in divided injections. Inject slowly, deep into the deltoid or gluteal muscle.

INVEGA TRINZA^{™} must be administered using only the thin wall needles that are provided in the INVEGA TRINZA^{™} pack. Do not use needles from the 1-month paliperidone palmitate extended-release injectable suspension pack or other commercially-available needles to reduce the risk of blockage.

### Deltoid Injection

The recommended needle size for administration of INVEGA TRINZA^{™} into the deltoid muscle is determined by the patient's weight:
- For patients weighing less than 90 kg, the 1-inch, 22 gauge thin wall needle is recommended.
- For patients weighing 90 kg or more, the 1½-inch, 22 gauge thin wall needle is recommended.

Administer into the center of the deltoid muscle. Deltoid injections should be alternated between the two deltoid muscles.

### Gluteal Injection

Regardless of patient weight, the recommended needle size for administration of INVEGA TRINZA^{™} into the gluteal muscle is the 1½-inch, 22 gauge thin wall needle. Administer into the upper-outer quadrant of the gluteal muscle. Gluteal injections should be alternated between the two gluteal muscles.

### Incomplete Administration

To avoid an incomplete administration of INVEGA TRINZA^{™}, ensure that the prefilled syringe is **shaken vigorously for at least 15 seconds within 5 minutes prior to administration to ensure a homogeneous suspension and ensure the needle does not get clogged during injection** *[see Dosage and Administration (2.8)].*

However, in the event of an incompletely administered dose, do not re-inject the dose remaining in the syringe and do not administer another dose of INVEGA TRINZA^{™}. Closely monitor and treat the patient with oral supplementation as clinically appropriate until the next scheduled 3-month injection of INVEGA TRINZA^{™}.

### 2.2 Schizophrenia

### Adults

INVEGA TRINZA^{™} is to be used only after INVEGA SUSTENNA^{®} (1-month paliperidone palmitate extended-release injectable suspension) has been established as adequate treatment for at least four months. In order to establish a consistent maintenance dose, it is recommended that the last two doses of INVEGA SUSTENNA^{®} be the same dosage strength before starting INVEGA TRINZA^{™}.

Initiate INVEGA TRINZA^{™} when the next 1-month paliperidone palmitate dose is scheduled with an INVEGA TRINZA^{™} dose based on the previous 1-month injection dose, using the equivalent 3.5-fold higher dose as shown in Table 1. INVEGA TRINZA^{™} may be administered up to 7 days before or after the monthly time point of the next scheduled paliperidone palmitate 1-month dose.

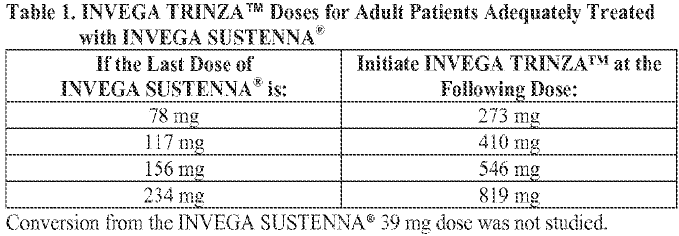

Following the initial INVEGA TRINZA^{™} dose, INVEGA TRINZA^{™} should be administered every 3 months. If needed, dose adjustment can be made every 3 months in increments within the range of 273 mg to 819 mg based on individual patient tolerability and/or efficacy. Due to the long-acting nature of INVEGA TRINZA^{™}, the patient's response to an adjusted dose may not be apparent for several months *[see Clinical Pharmacology (12.3)].*

### 2.3 Missed Doses

### Dosing Window

Missing doses of INVEGA TRINZA^{™} should be avoided. If necessary, patients may be given the injection up to 2 weeks before or after the 3-month time point.

### Missed Dose 3½ Months to 4 Months Since Last Injection

If more than 3½ months (up to but less than 4 months) have elapsed since the last injection of INVEGA TRINZA^{™}, the previously administered INVEGA TRINZA^{™} dose should be administered as soon as possible, then continue with the 3-month injections following this dose.

### Missed Dose 4 Months to 9 Months Since Last Injection

If 4 months up to and including 9 months have elapsed since the last injection of INVEGA TRINZA^{™}, do NOT administer the next dose of INVEGA TRINZA^{™}. Instead, use the re-initiation regimen shown in Table 2.

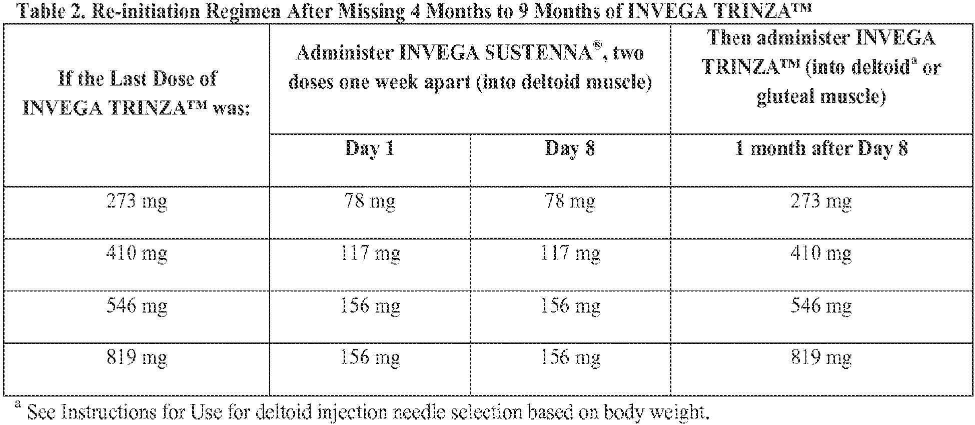

### Missed Dose Longer than 9 Months Since Last Injection

If more than 9 months have elapsed since the last injection of INVEGA TRINZA^{™}, re-initiate treatment with the 1-month paliperidone palmitate extended-release injectable suspension as described in the prescribing information for that product. INVEGA TRINZA^{™} can then be resumed after the patient has been adequately treated with the 1-month paliperidone palmitate extended-release injectable suspension for at least 4 months.

### 2.4 Use with Risperidone or with Oral Paliperidone

Since paliperidone is the major active metabolite of risperidone, caution should be exercised when INVEGA TRINZA^{™} is coadministered with risperidone or oral paliperidone for extended periods of time. Safety data involving concomitant use of INVEGA TRINZA^{™} with other antipsychotics is limited.

### 2.5 Dosage Adjustment in Renal Impairment

INVEGA TRINZA^{™} has not been systematically studied in patients with renal impairment *[see Clinical Pharmacology (12.3)].* For patients with mild renal impairment (creatinine clearance ≥ 50 mL/min to < 80 mL/min [Cockcroft-Gault Formula], adjust dosage and stabilize the patient using the 1-month paliperidone palmitate extended-release injectable suspension, then transition to INVEGA TRINZA^{™} *[see Table 1*, *Dosage and Administration (2.2)]. [See also Use in*

### Specific Populations (8.6) and Clinical Pharmacology (12.3)]

INVEGA TRINZA^{™} is not recommended in patients with moderate or severe renal impairment (creatinine clearance < 50 mL/min) *[see Use in Specific Populations (8.6) and Clinical Pharmacology (12.3)].*

### 2.6 Switching from INVEGA TRINZA^{™} to the 1-Month Paliperidone Palmitate Extended-Release Injectable Suspension

For switching from INVEGA TRINZA^{™} to INVEGA SUSTENNA^{®} (1-month paliperidone palmitate extended-release injectable suspension), the 1-month paliperidone palmitate extended-release injectable suspension should be started 3 months after the last INVEGA TRINZA^{™} dose, using the equivalent 3.5-fold lower dose as shown in Table 3. The 1-month paliperidone palmitate extended-release injectable suspension should then continue, dosed at monthly intervals.

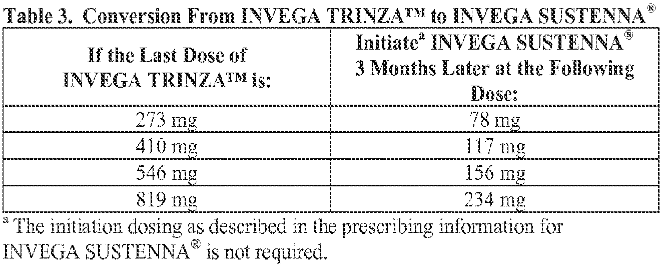

### 2.7 Switching from INVEGA TRINZA^{™} to Oral Paliperidone Extended-Release Tablets

For switching from INVEGA TRINZA^{™} to oral paliperidone extended-release tablets, the daily dosing of the paliperidone extended-release tablets should be started 3 months after the last INVEGA TRINZA^{™} dose and transitioned over the next several months following the last INVEGA TRINZA^{™} dose as described in Table 4. Table 4 provides dose conversion regimens to allow patients previously stabilized on different doses of INVEGA TRINZA^{™} to attain similar paliperidone exposure with once daily paliperidone extended-release tablets.

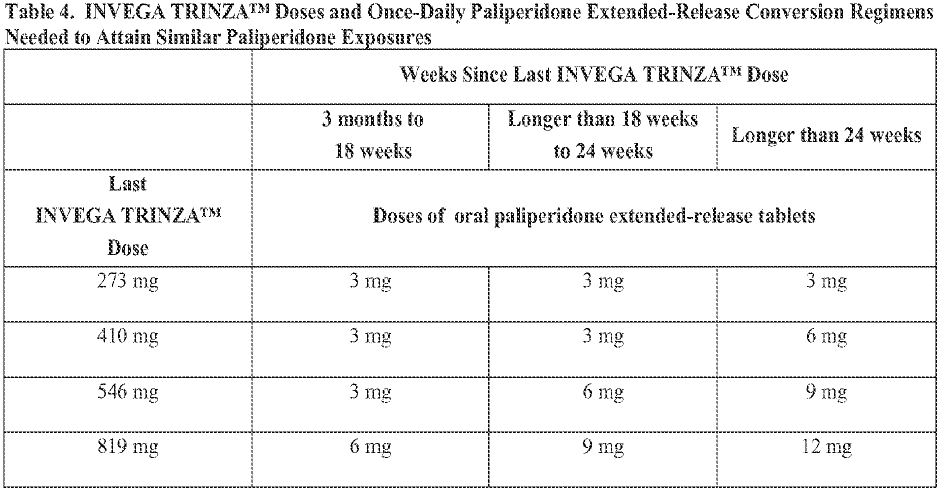

### 2.8 Instructions for Use

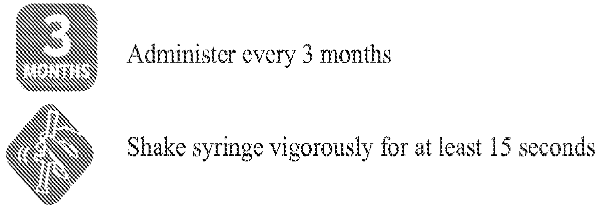

### For intramuscular injection only. Do not administer by any other route.

### Important

INVEGA TRINZA^{™} should be administered by a healthcare professional as a single injection. **DO NOT** divide dose into multiple injections.

INVEGA TRINZA^{™} is intended for intramuscular use only. Inject slowly, deep into the muscle taking care to avoid injection into a blood vessel.

Read complete instructions prior to use.

### Dosing

This medication should be administered **once every 3 months.**

### Preparation

Peel off tab label from the syringe and place in patient record.

INVEGA TRINZA^{™} requires longer and more vigorous shaking than INVEGA SUSTENNA^{®} (1-month paliperidone palmitate extended-release injectable suspension). Shake the syringe vigorously, with the syringe tip pointing up. for **at least 15 seconds within 5 minutes prior to administration** (see Step 2).

### Thin Wall Safety Needle Selection

Thin wall safety needles are designed to be used with INVEGA TRINZA^{™}. Therefore, it is important to **only use the needles provided in the INVEGA TRINZA^{™} kit.**

### Dose pack contents

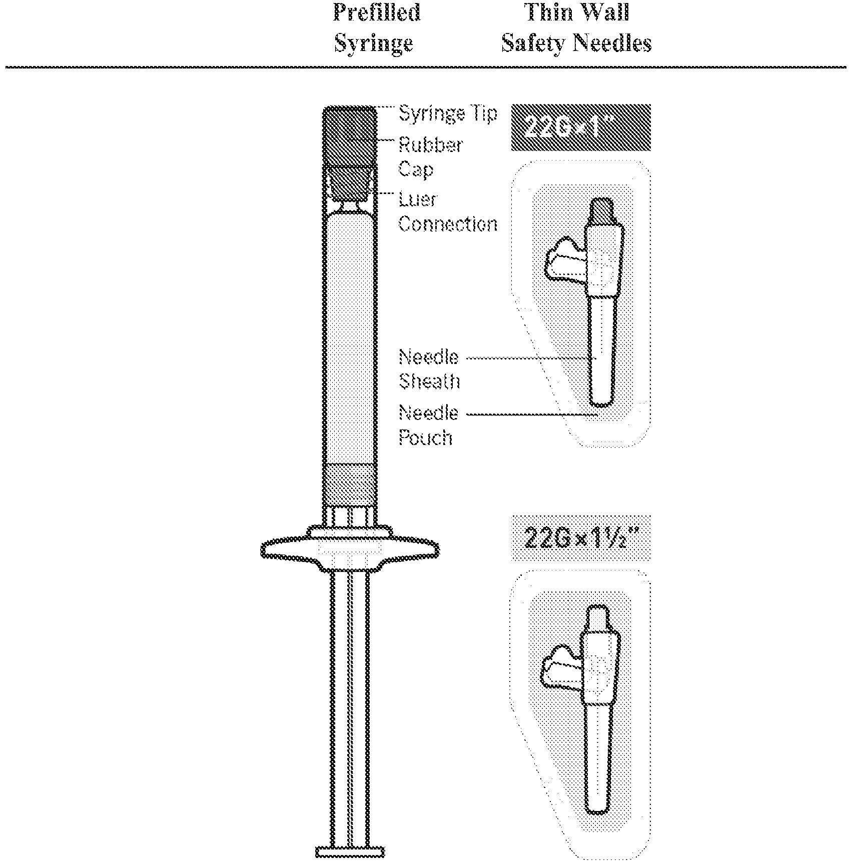

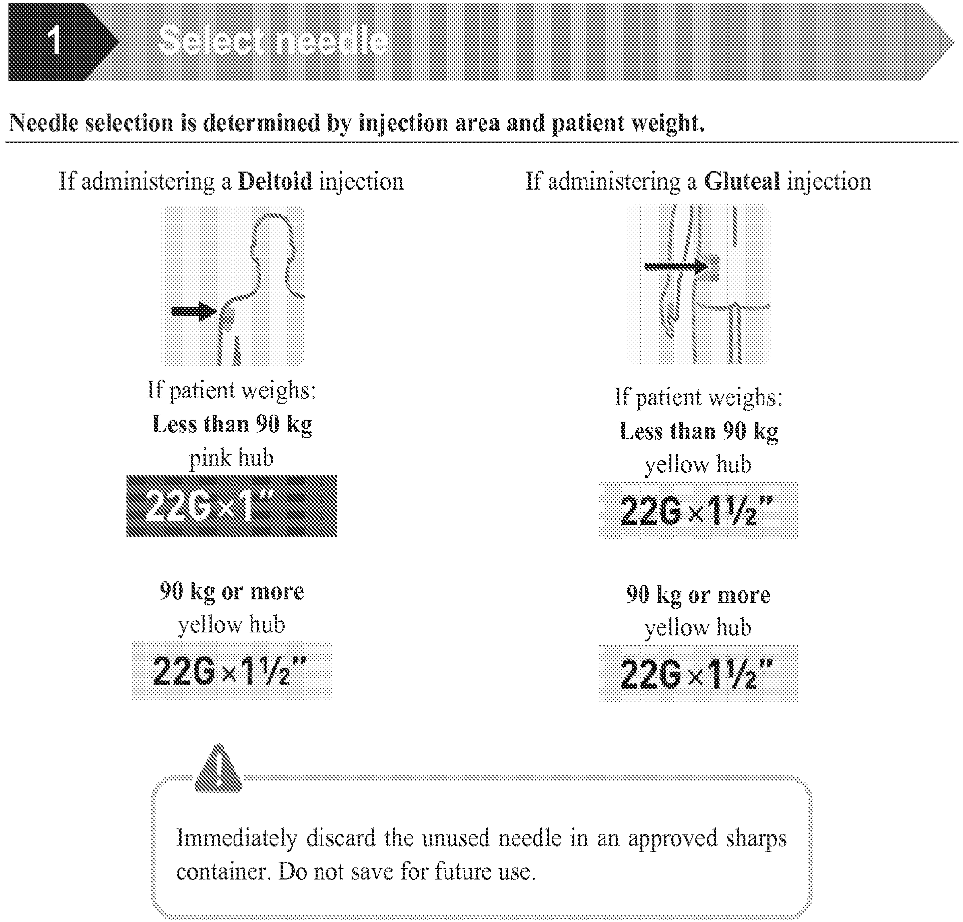

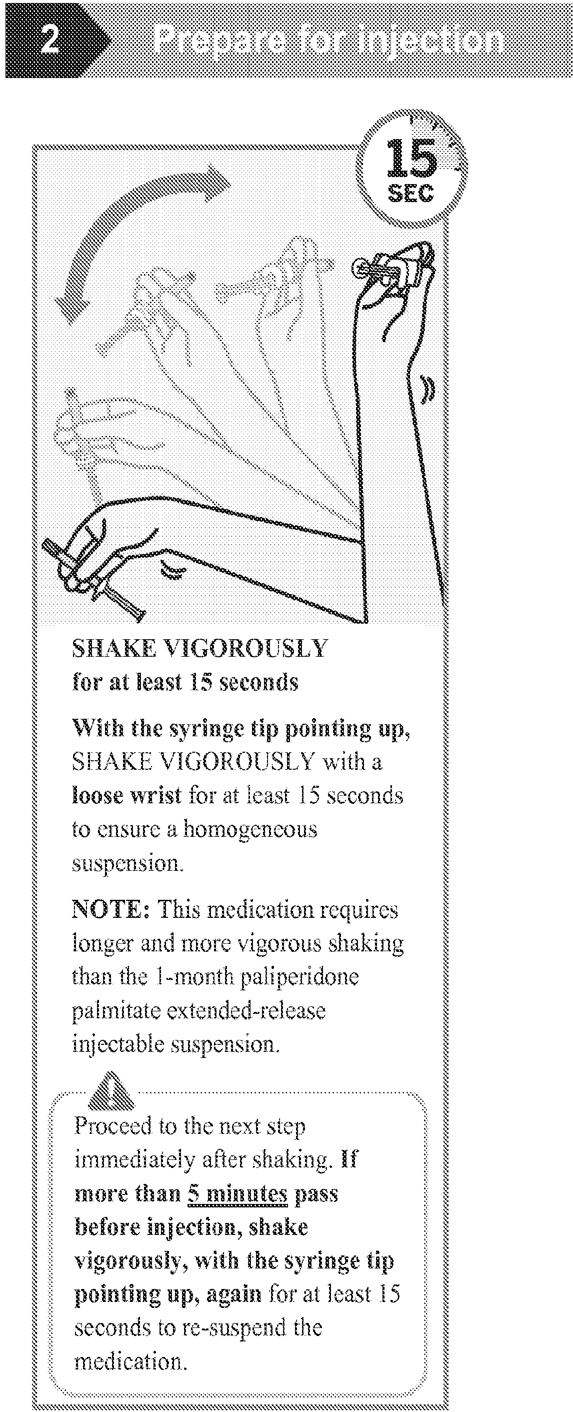

### Check suspension

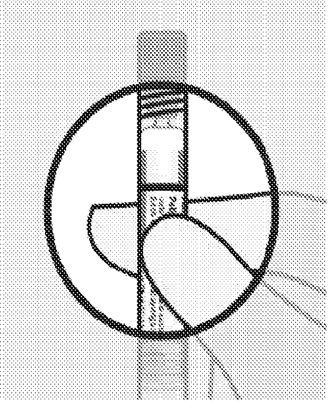

After shaking the syringe for at least 15 seconds, check the liquid in the viewing window.

The suspension should appear uniform and milky white in color.

It is also normal to see small air bubbles.

### Open needle pouch and remove cap

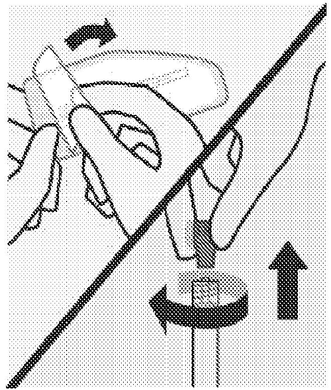

First, open needle pouch by peeling the cover back half way. Place on a clean surface.

Then, holding the syringe upright, twist and pull the rubber cap to remove.

### Grasp needle pouch

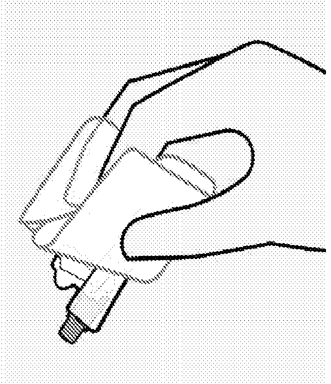

Fold back needle cover and plastic tray. Then, firmly grasp the needle sheath through the pouch, as shown.

### Attach needle

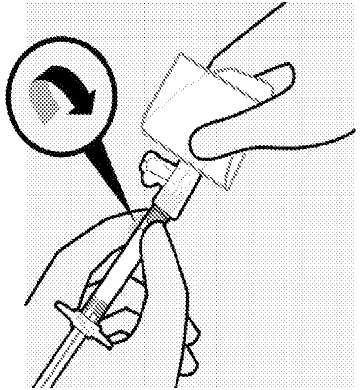

With your other hand, hold the syringe by the luer connection and attach it to the safety needle with a gentle clockwise twisting motion.

**Do not** remove the pouch until the syringe and needle are securely attached.

### Remove needle sheath

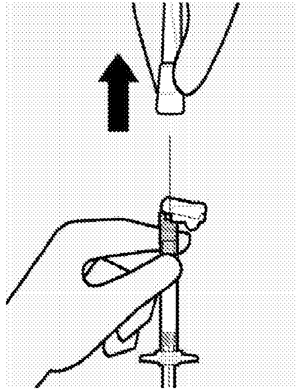

Pull the needle sheath away from the needle in a straight motion.

**Do not** twist the sheath, as this may loosen the needle from the syringe.

### Remove air bubbles

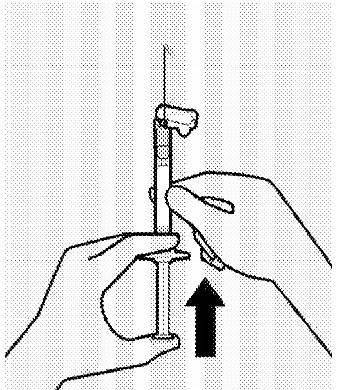

Hold the syringe upright and tap gently to make any air bubbles rise to the top.

Remove air by pressing the plunger rod upward carefully until a drop of liquid comes out of the needle tip.

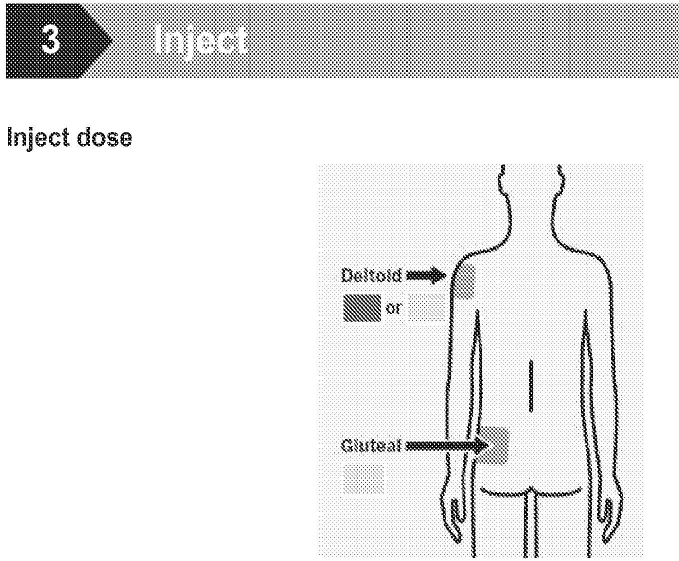

**Slowly inject the entire contents of the syringe** intramuscularly, deep into the selected deltoid or gluteal muscle.

### Do not administer by any other route.

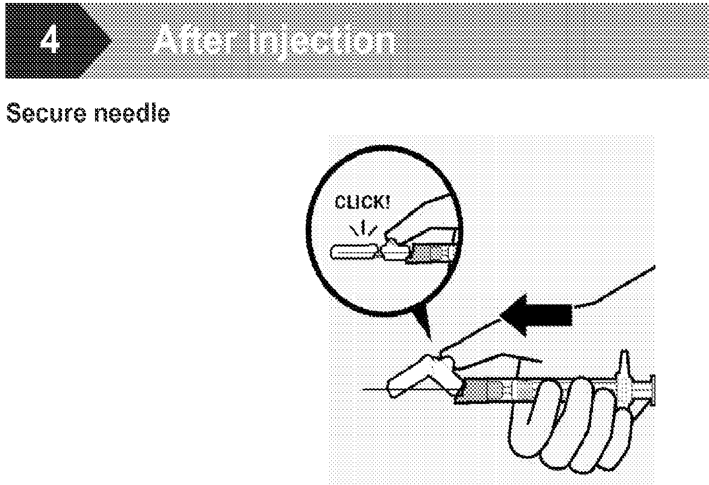

After the injection is complete, use your thumb or a flat surface to secure the needle in the safety device. The needle is secure when a "click" sound is heard.

### Dispose properly

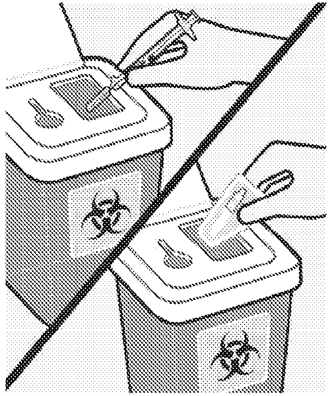

Dispose of the syringe and unused needle in an approved sharps container.

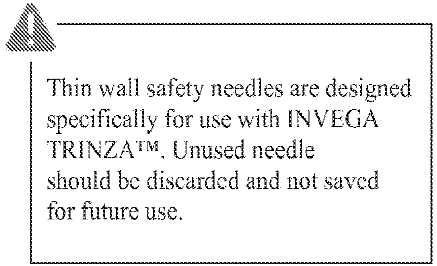

### 3 DOSAGE FORMS AND STRENGTHS

INVEGA TRINZA^{™} is available as a white to off-white aqueous extended-release injectable suspension for intramuscular injection in dose strengths of 273 mg, 410 mg, 546 mg, and 819 mg paliperidone palmitate.

### 4 CONTRAINDICATIONS

INVEGA TRINZA^{™} is contraindicated in patients with a known hypersensitivity to either paliperidone or risperidone, or to any of the excipients in the INVEGA TRINZA^{™} formulation. Hypersensitivity reactions, including anaphylactic reactions and angioedema, have been observed in patients treated with risperidone and paliperidone. Paliperidone palmitate is converted to paliperidone, which is a metabolite of risperidone.

### 5 WARNINGS AND PRECAUTIONS

### 5.1 Increased Mortality in Elderly Patients with Dementia-Related Psychosis

Elderly patients with dementia-related psychosis treated with antipsychotic drugs are at an increased risk of death. Analyses of 17 placebo-controlled trials (modal duration of 10 weeks), largely in patients taking atypical antipsychotic drugs, revealed a risk of death in drug-treated patients of between 1.6 to 1.7 times the risk of death in placebo-treated patients. Over the course of a typical 10-week controlled trial, the rate of death in drug-treated patients was about 4.5%, compared to a rate of about 2.6% in the placebo group. Although the causes of death were varied, most of the deaths appeared to be either cardiovascular (e.g., heart failure, sudden death) or infectious (e.g., pneumonia) in nature. Observational studies suggest that, similar to atypical antipsychotic drugs, treatment with conventional antipsychotic drugs may increase mortality. The extent to which the findings of increased mortality in observational studies may be attributed to the antipsychotic drug as opposed to some characteristic(s) of the patients is not clear. INVEGA TRINZA^{™} is not approved for the treatment of patients with dementia-related psychosis *[see Boxed Warning and Warnings and Precautions (5.2)].*

### 5.2 Cerebrovascular Adverse Reactions, Including Stroke, in Elderly Patients with Dementia-Related Psychosis

In placebo-controlled trials with risperidone, aripiprazole, and olanzapine in elderly subjects with dementia, there was a higher incidence of cerebrovascular adverse reactions (cerebrovascular accidents and transient ischemic attacks) including fatalities compared to placebo-treated subjects. No studies have been conducted with oral paliperidone, the 1-month paliperidone palmitate extended-release injectable suspension, or INVEGA TRINZA^{™} in elderly patients with dementia. These medications are not approved for the treatment of patients with dementia-related psychosis *[see Boxed Warning and Warnings and Precautions (5.1)].*

### 5.3 Neuroleptic Malignant Syndrome

A potentially fatal symptom complex sometimes referred to as Neuroleptic Malignant Syndrome (NMS) has been reported in association with antipsychotic drugs, including paliperidone. Clinical manifestations of NMS are hyperpyrexia, muscle rigidity, altered mental status, and evidence of autonomic instability (irregular pulse or blood pressure, tachycardia, diaphoresis, and cardiac dysrhythmia). Additional signs may include elevated creatine phosphokinase, myoglobinuria (rhabdomyolysis), and acute renal failure.

The diagnostic evaluation of patients with this syndrome is complicated. In arriving at a diagnosis, it is important to identify cases in which the clinical presentation includes both serious medical illness (e.g., pneumonia, systemic infection, etc.) and untreated or inadequately treated extrapyramidal signs and symptoms (EPS). Other important considerations in the differential diagnosis include central anticholinergic toxicity, heat stroke, drug fever, and primary central nervous system pathology.

The management of NMS should include: (1) immediate discontinuation of antipsychotic drugs and other drugs not essential to concurrent therapy; (2) intensive symptomatic treatment and medical monitoring; and (3) treatment of any concomitant serious medical problems for which specific treatments are available. Consideration should be given to the long-acting nature of INVEGA TRINZA^{™}. There is no general agreement about specific pharmacological treatment regimens for uncomplicated NMS.

If a patient appears to require antipsychotic drug treatment after recovery from NMS, reintroduction of drug therapy should be closely monitored, since recurrences of NMS have been reported.

### 5.4 QT Prolongation

Paliperidone causes a modest increase in the corrected QT (QTc) interval. The use of paliperidone should be avoided in combination with other drugs that are known to prolong QTc including Class 1A (e.g., quinidine, procainamide) or Class III (e.g., amiodarone, sotalol) antiarrhythmic medications, antipsychotic medications (e.g., chlorpromazine, thioridazine), antibiotics (e.g., gatifloxacin, moxifloxacin), or any other class of medications known to prolong the QTc interval. Paliperidone should also be avoided in patients with congenital long QT syndrome and in patients with a history of cardiac arrhythmias.

Certain circumstances may increase the risk of the occurrence of Torsades de pointes and/or sudden death in association with the use of drugs that prolong the QTc interval, including (1) bradycardia; (2) hypokalemia or hypomagnesemia; (3) concomitant use of other drugs that prolong the QTc interval; and (4) presence of congenital prolongation of the QT interval.

The effects of paliperidone on the QT interval were evaluated in a double-blind, active-controlled (moxifloxacin 400 mg single dose), multicenter Thorough QT study with oral paliperidone in adult patients, and in four fixed-dose efficacy studies and one maintenance study of the 1-month paliperidone palmitate injectable product.

In the Thorough QT study (n=141), the 8 mg dose of immediate-release oral paliperidone (n=50) showed a mean placebo-subtracted increase from baseline in QTcLD (QT interval corrected for heart rate using the population specified linear derived method) of 12.3 msec (90% CI: 8.9; 15.6) on day 8 at 1.5 hours post-dose. The mean steady-state peak plasma concentration for this 8 mg dose of paliperidone immediate release (C_{max ss}=113 ng/mL) was approximately 2-fold the exposure with the maximum recommended 819 mg dose of INVEGA TRINZA^{™} administered in the deltoid muscle (predicted median C_{max ss}=56 ng/mL). In this same study, a 4 mg dose of the immediate-release oral formulation of paliperidone. for which C_{max ss}=35 ng/mL, showed an increased placebo-subtracted QTcLD of 6.8 msec (90% CI: 3.6; 10.1) on day 2 at 1.5 hours post-dose.

In the four fixed-dose efficacy studies of the 1-month paliperidone palmitate injectable product, no subject had a change in QTcLD exceeding 60 msec and no subject had a QTcLD value of > 500 msec at any time point. In the maintenance study, no subject had a QTcLD change > 60 msec, and one subject had a QTcLD value of 507 msec (Bazett's QT corrected interval [QTcB] value of 483 msec); this latter subject also had a heart rate of 45 beats per minute.

In the long-term maintenance trial of INVEGA TRINZA^{™} in subjects with schizophrenia, an increase in QTcLD exceeding 60 msec was observed in 1 subject (< 1%) in the open-label phase, no subject had an increase in QTcLD exceeding 60 msec after treatment with INVEGA TRINZA^{™} in the double-blind phase, and no subject had a QTcLD value of > 480 msec at any point in the study.

### 5.5 Tardive Dyskinesia

A syndrome of potentially irreversible, involuntary, dyskinetic movements may develop in patients treated with antipsychotic drugs. Although the prevalence of the syndrome appears to be highest among the elderly, especially elderly women, it is impossible to predict which patients will develop the syndrome. Whether antipsychotic drug products differ in their potential to cause tardive dyskinesia is unknown.

The risk of developing tardive dyskinesia and the likelihood that it will become irreversible appear to increase as the duration of treatment and the total cumulative dose of antipsychotic drugs administered to the patient increase, but the syndrome can develop after relatively brief treatment periods at low doses, although this is uncommon.

There is no known treatment for established tardive dyskinesia, although the syndrome may remit, partially or completely, if antipsychotic treatment is withdrawn. Antipsychotic treatment itself may suppress (or partially suppress) the signs and symptoms of the syndrome and may thus mask the underlying process. The effect of symptomatic suppression on the long-term course of the syndrome is unknown.

Given these considerations, INVEGA TRINZA^{™} should be prescribed in a manner that is most likely to minimize the occurrence of tardive dyskinesia. Chronic antipsychotic treatment should generally be reserved for patients who suffer from a chronic illness that is known to respond to antipsychotic drugs. In patients who do require chronic treatment, the smallest dose and the shortest duration of treatment producing a satisfactory clinical response should be sought. The need for continued treatment should be reassessed periodically.

If signs and symptoms of tardive dyskinesia appear in a patient treated with INVEGA TRINZA^{™}, drug discontinuation should be considered. Consideration should be given to the long-acting nature of INVEGA TRINZA^{™}. However, some patients may require treatment with INVEGA TRINZA^{™} despite the presence of the syndrome.

### 5.6 Metabolic Changes

Atypical antipsychotic drugs have been associated with metabolic changes that may increase cardiovascular/cerebrovascular risk. These metabolic changes include hyperglycemia, dyslipidemia, and body weight gain. While all of the drugs in the class have been shown to produce some metabolic changes, each drug has its own specific risk profile.

### Hyperglycemia and Diabetes Mellitus

Hyperglycemia and diabetes mellitus, in some cases extreme and associated with ketoacidosis or hyperosmolar coma or death, have been reported in patients treated with all atypical antipsychotics. These cases were, for the most part, seen in post-marketing clinical use and epidemiologic studies, not in clinical trials. Hyperglycemia and diabetes have been reported in trial subjects treated with INVEGA TRINZA^{™}. Assessment of the relationship between atypical antipsychotic use and glucose abnormalities is complicated by the possibility of an increased background risk of diabetes mellitus in patients with schizophrenia and the increasing incidence of diabetes mellitus in the general population. Given these confounders, the relationship between atypical antipsychotic use and hyperglycemia-related adverse events is not completely understood. However, epidemiological studies suggest an increased risk of hyperglycemia-related adverse reactions in patients treated with the atypical antipsychotics.

Patients with an established diagnosis of diabetes mellitus who are started on atypical antipsychotics should be monitored regularly for worsening of glucose control. Patients with risk factors for diabetes mellitus (e.g., obesity, family history of diabetes) who are starting treatment with atypical antipsychotics should undergo fasting blood glucose testing at the beginning of treatment and periodically during treatment. Any patient treated with atypical antipsychotics should be monitored for symptoms of hyperglycemia including polydipsia, polyuria, polyphagia, and weakness. Patients who develop symptoms of hyperglycemia during treatment with atypical antipsychotics should undergo fasting blood glucose testing. In some cases, hyperglycemia has resolved when the atypical antipsychotic was discontinued; however, some patients required continuation of anti-diabetic treatment despite discontinuation of the suspect drug.

Data from the long-term maintenance trial with INVEGA TRINZA^{™} in subjects with schizophrenia are presented in Table 5.

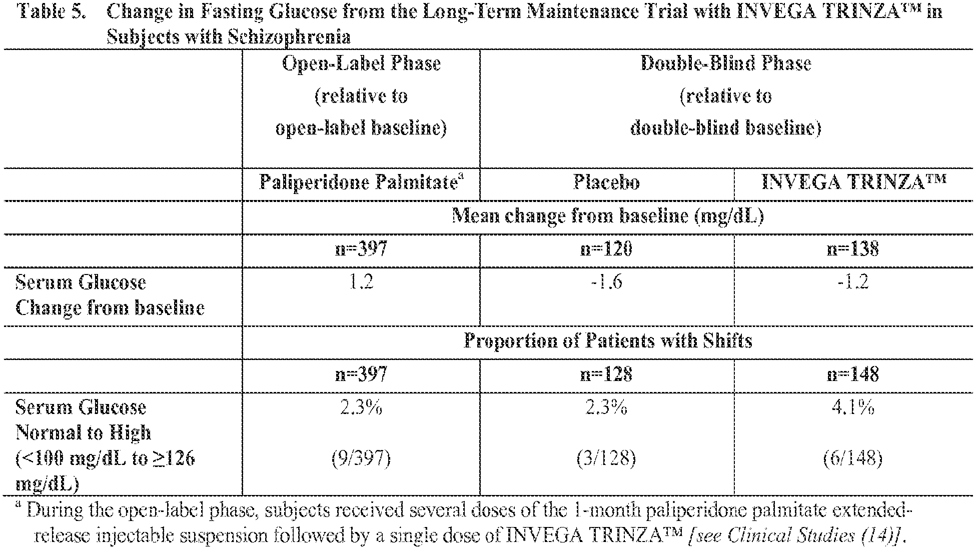

### Dyslipidemia

Undesirable alterations in lipids have been observed in patients treated with atypical antipsychotics.

Data from the long-term maintenance trial with INVEGA TRINZA^{™} in subjects with schizophrenia are presented in Table 6.

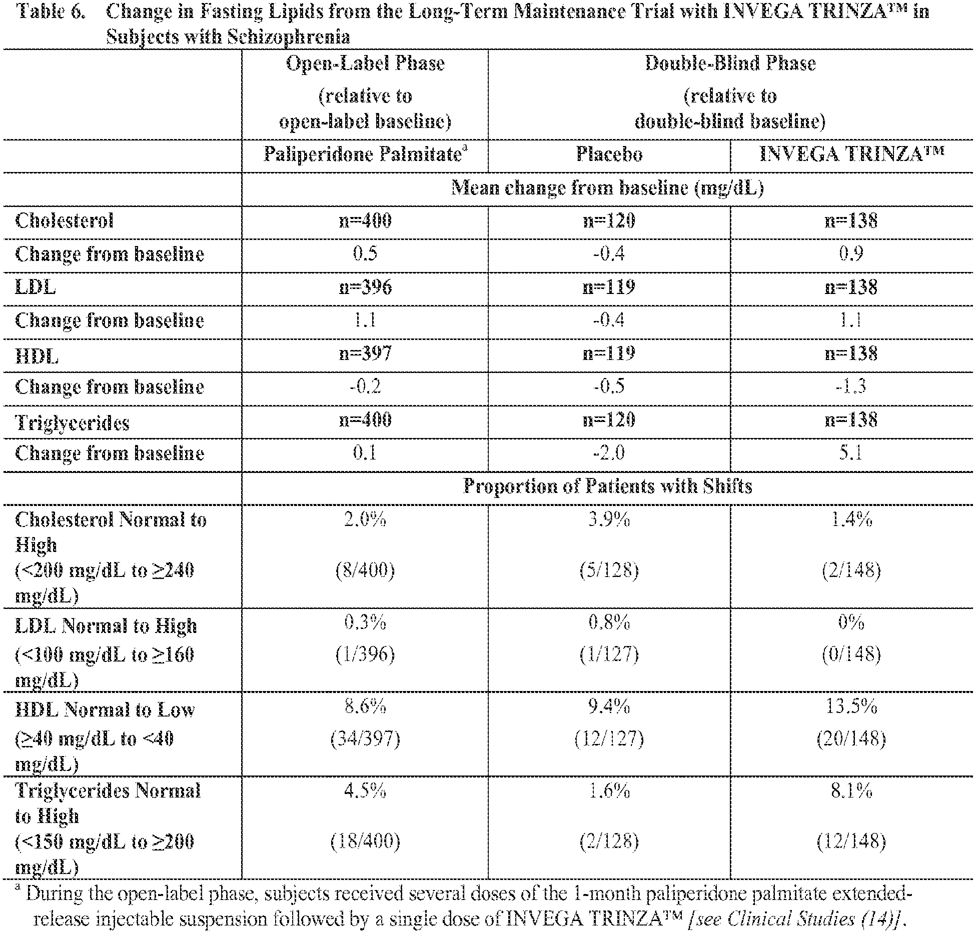

### Weight Gain

Weight gain has been observed with atypical antipsychotic use. Clinical monitoring of weight is recommended.

Data on mean changes in body weight and the proportion of subjects meeting a weight gain criterion of ≥ 7% of body weight from the long-term maintenance trial with INVEGA TRINZA^{™} in subjects with schizophrenia are presented in Table 7.

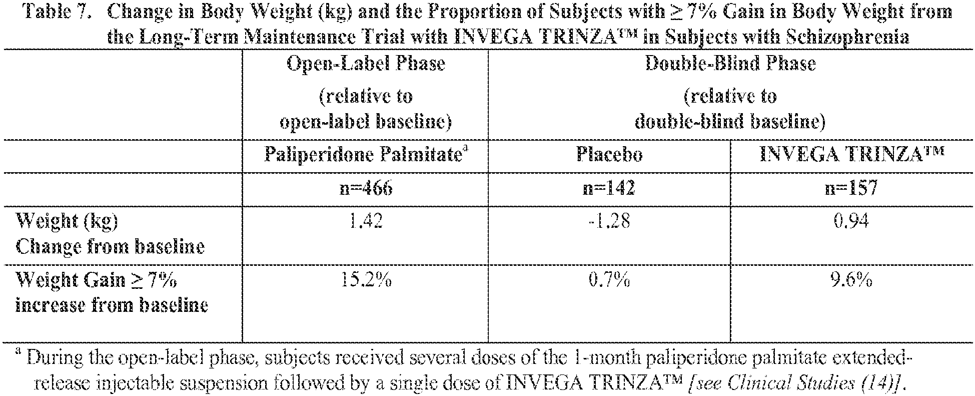

### 5.7 Orthostatic Hypotension and Syncope

Paliperidone can induce orthostatic hypotension and syncope in some patients because of its alpha-adrenergic blocking activity. In the long-term maintenance trial, syncope was reported in < 1% (1/506) of subjects treated with the 1-month paliperidone palmitate extended-release injectable suspension during the open-label phase; there were no cases reported during the double-blind phase in either treatment group. In the long-term maintenance trial, orthostatic hypotension was reported as an adverse event by < 1% (1/506) of subjects treated with the 1-month paliperidone palmitate extended-release injectable suspension and < 1% (1/379) of subjects after receiving a single-dose of INVEGA TRINZA^{™} during the open-label phase; there were no cases reported during the double-blind phase in either treatment group.

INVEGA TRINZA^{™} should be used with caution in patients with known cardiovascular disease (e.g., heart failure, history of myocardial infarction or ischemia, conduction abnormalities), cerebrovascular disease, or conditions that predispose the patient to hypotension (e.g., dehydration, hypovolemia, and treatment with antihypertensive medications). Monitoring of orthostatic vital signs should be considered in patients who are vulnerable to hypotension.

### 5.8 Leukopenia, Neutropenia, and Agranulocytosis

In clinical trial and/or postmarketing experience, events of leukopenia and neutropenia have been reported temporally related to antipsychotic agents, including INVEGA TRINZA^{™}. Agranulocytosis has also been reported.

Possible risk factors for leukopenia/neutropenia include pre-existing low white blood cell count (WBC)/absolute neutrophil count (ANC) and history of drug-induced leukopenia/neutropenia. In patients with a history of a clinically significant low WBC/ANC or a drug-induced leukopenia/neutropenia, perform a complete blood count (CBC) frequently during the first few months of therapy. In such patients, consider discontinuation of INVEGA TRINZA^{™} at the first sign of a clinically significant decline in WBC in the absence of other causative factors.

Monitor patients with clinically significant neutropenia for fever or other symptoms or signs of infection and treated promptly if such symptoms or signs occur. Discontinue INVEGA TRINZA^{™} in patients with severe neutropenia (absolute neutrophil count <1000/mm³) follow their WBC until recovery.

### 5.9 Hyperprolactinemia

Like other drugs that antagonize dopamine D₂ receptors, paliperidone elevates prolactin levels and the elevation persists during chronic administration. Paliperidone has a prolactin-elevating effect similar to that seen with risperidone, a drug that is associated with higher levels of prolactin than other antipsychotic drugs.

Hyperprolactinemia, regardless of etiology, may suppress hypothalamic GnRH, resulting in reduced pituitary gonadotrophin secretion. This, in turn, may inhibit reproductive function by impairing gonadal steroidogenesis in both female and male patients. Galactorrhea, amenorrhea, gynecomastia, and impotence have been reported in patients receiving prolactin-elevating compounds. Long-standing hyperprolactinemia when associated with hypogonadism may lead to decreased bone density in both female and male subjects.

Tissue culture experiments indicate that approximately one-third of human breast cancers are prolactin dependent *in vitro*, a factor of potential importance if the prescription of these drugs is considered in a patient with previously detected breast cancer. An increase in the incidence of pituitary gland, mammary gland, and pancreatic islet cell neoplasia (mammary adenocarcinomas, pituitary and pancreatic adenomas) was observed in the risperidone carcinogenicity studies conducted in mice and rats *[see Nonclinical Toxicology (13.1)].* Neither clinical studies nor epidemiologic studies conducted to date have shown an association between chronic administration of this class of drugs and tumorigenesis in humans, but the available evidence is too limited to be conclusive.

In a long-term maintenance trial of INVEGA TRINZA^{™}, elevations of prolactin to above the reference range (>13.13 ng/mL in males and >26.72 ng/mL in females) relative to open-label baseline at any time during the double-blind phase were noted in a higher percentage of males in the INVEGA TRINZA^{™} group than in the placebo group (46% vs. 25%) and in a higher percentage of females in the INVEGA TRINZA^{™} group than in the placebo group (32% vs. 15%). During the double-blind phase, 1 female (2.4%) in the INVEGA TRINZA^{™} group experienced an adverse reaction of amenorrhea, while no potentially prolactin-related adverse reactions were noted among females in the placebo group. There were no potentially prolactin-related adverse reactions among males in either group.

Prior to the double-blind phase (during the 29-week open-label phase of the long-term maintenance trial), the mean (SD) serum prolactin values at baseline in males (N=368) were 17.1 (13.55) ng/mL and 51.6 (40.85) ng/mL in females (N=122). Twelve weeks after a single injection of INVEGA TRINZA^{™} at the end of the open-label phase, mean (SD) prolactin values were 25.8 (13.49) ng/mL in males (N=322) and 70.6 (40.23) ng/mL in females (N=107). During the open-label phases 27% of females and 42% of males experienced elevations of prolactin above the reference range relative to baseline, and a higher proportion of females experienced potentially prolactin-related adverse reactions compared to males (7.9% vs. 3.7%). Amenorrhea (4.7%) and galactorrhea (3.1%) were the most commonly observed (≥3%) potentially prolactin-related adverse reactions in females. Among males in the open-label phase, no potentially prolactin-related adverse reaction was observed with a rate greater than 3%.

### 5.10 Potential for Cognitive and Motor Impairment

Somnolence, sedation, and dizziness were reported as adverse reactions in subjects treated with INVEGA TRINZA^{™} *[see Adverse Reactions (6.1)].* Antipsychotics, including INVEGA TRINZA^{™}, have the potential to impair judgment, thinking, or motor skills. Patients should be cautioned about performing activities requiring mental alertness, such as operating hazardous machinery or operating a motor vehicle, until they are reasonably certain that paliperidone therapy does not adversely affect them.

### 5.11 Seizures

In the long-term maintenance trial there were no reports of seizures or convulsions. In the pivotal clinical studies with the 1-month paliperidone palmitate extended-release injectable suspension which included four fixed-dose, double-blind, placebo-controlled studies in subjects with schizophrenia, <1% (1/1293) of subjects treated with the 1-month injection experienced an adverse event of convulsion compared with <1% (1/510) of placebo-treated subjects who experienced an adverse event of grand mal convulsion.

Like other antipsychotic drugs, INVEGA TRINZA^{™} should be used cautiously in patients with a history of seizures or other conditions that potentially lower the seizure threshold. Conditions that lower the seizure threshold may be more prevalent in patients 65 years or older.

### 5.12 Dysphagia

Esophageal dysmotility and aspiration have been associated with antipsychotic drug use. INVEGA TRINZA^{™} and other antipsychotic drugs should be used cautiously in patients at risk for aspiration pneumonia.

### 5.13 Priapism

Drugs with alpha-adrenergic blocking effects have been reported to induce priapism. Although no cases of priapism have been reported in clinical trials with INVEGA TRINZA^{™}, priapism has been reported with oral paliperidone during postmarketing surveillance. Severe priapism may require surgical intervention.

### 5.14 Disruption of Body Temperature Regulation

Disruption of the body's ability to reduce core body temperature has been attributed to antipsychotic agents. Appropriate care is advised when prescribing INVEGA TRINZA^{™} to patients who will be experiencing conditions which may contribute to an elevation in core body temperature, e.g., exercising strenuously, exposure to extreme heat, receiving concomitant medication with anticholinergic activity, or being subject to dehydration.

### 6 ADVERSE REACTIONS

The following are discussed in more detail in other sections of the labeling:
- Increased mortality in elderly patients with dementia-related psychosis *[see Boxed Warning and Warnings and Precautions (5.1)]*
- Cerebrovascular adverse reactions, including stroke, in elderly patients with dementia-related psychosis *[see Warnings and Precautions (5.2)]*
- Neuroleptic malignant syndrome *[see Warnings and Precautions (5.3)]*
- QT prolongation *[see Warnings and Precautions (5.4)]*
- Tardive dyskinesia *[see Warnings and Precautions (5.5)]*
- Metabolic changes *[see Warnings and Precautions (5.6)]*
- Orthostatic hypotension and syncope *[see Warnings and Precautions (5.7)]*
- Leukopenia, neutropenia, and agranulocytosis *[see Warnings and Precautions (5.8)]*
- Hyperprolactinemia *[see Warnings and Precautions (5.9)]*
- Potential for cognitive and motor impairment *[see Warnings and Precautions (5.10)]*
- Seizures *[see Warnings and Precautions (5.11)]*
- Dysphagia *[see Warnings and Precautions (5.12)]*
- Priapism *[see Warnings and Precautions (5.13)]*
- Disruption of body temperature regulation *[see Warnings and Precautions (5.14)]*

### 6.1 Clinical Trials Experience

Because clinical trials are conducted under widely varying conditions, adverse reaction rates observed in the clinical trials of a drug cannot be directly compared to rates in the clinical trials of another drug and may not reflect the rates observed in clinical practice.

### Patient Exposure

The data described in this section include data from two clinical trials. One is a long-term maintenance trial, in which 506 subjects with schizophrenia received several doses of the 1-month paliperidone palmitate extended-release injectable suspension during the open-label phase, of which 379 subjects continued to receive a single injection of INVEGA TRINZA^{™} during the open-label phase. and 160 subjects were subsequently randomized to receive at least one dose of INVEGA TRINZA^{™} and 145 subjects received placebo during the double-blind placebo-controlled phase. The mean (SD) duration of exposure during the double-blind phase was 150 (79) days in the placebo group and 175 (90) days in the INVEGA TRINZA^{™} group. The other is a Phase 1 study (N=308), which included patients with schizophrenia who received a single injection of INVEGA TRINZA^{™} concomitantly with other oral antipsychotics.

### Adverse Reactions in a Double-Blind, Placebo-Controlled (Long-Term Maintenance) Clinical Trial

**Commonly Observed Adverse Reactions:** The most common adverse reactions (incidence at least 5% in the open-label phase, or in the INVEGA TRINZA^{™} group and at least twice the incidence in the placebo group during the double-blind phase) were injection site reaction, weight increased, headache, upper respiratory tract infection, akathisia, and parkinsonism.

**Discontinuation of Treatment Due to Adverse Events:** The percentages of subjects who discontinued due to adverse events in the long-term maintenance trial were 5.1% during the open-label phase. During the double-blind phase, no INVEGA TRINZA^{™}-treated subject and one placebo-treated subject discontinued due to adverse events.

**Adverse Reactions Occurring at an Incidence of 2% or More in INVEGA TRINZA^{™}-Treated Patients:** The safety profile of INVEGA TRINZA^{™} was similar to that seen with the 1-month paliperidone extended-release injectable suspension. Table 8 lists the adverse reactions reported in a long-term maintenance trial in subjects with schizophrenia.

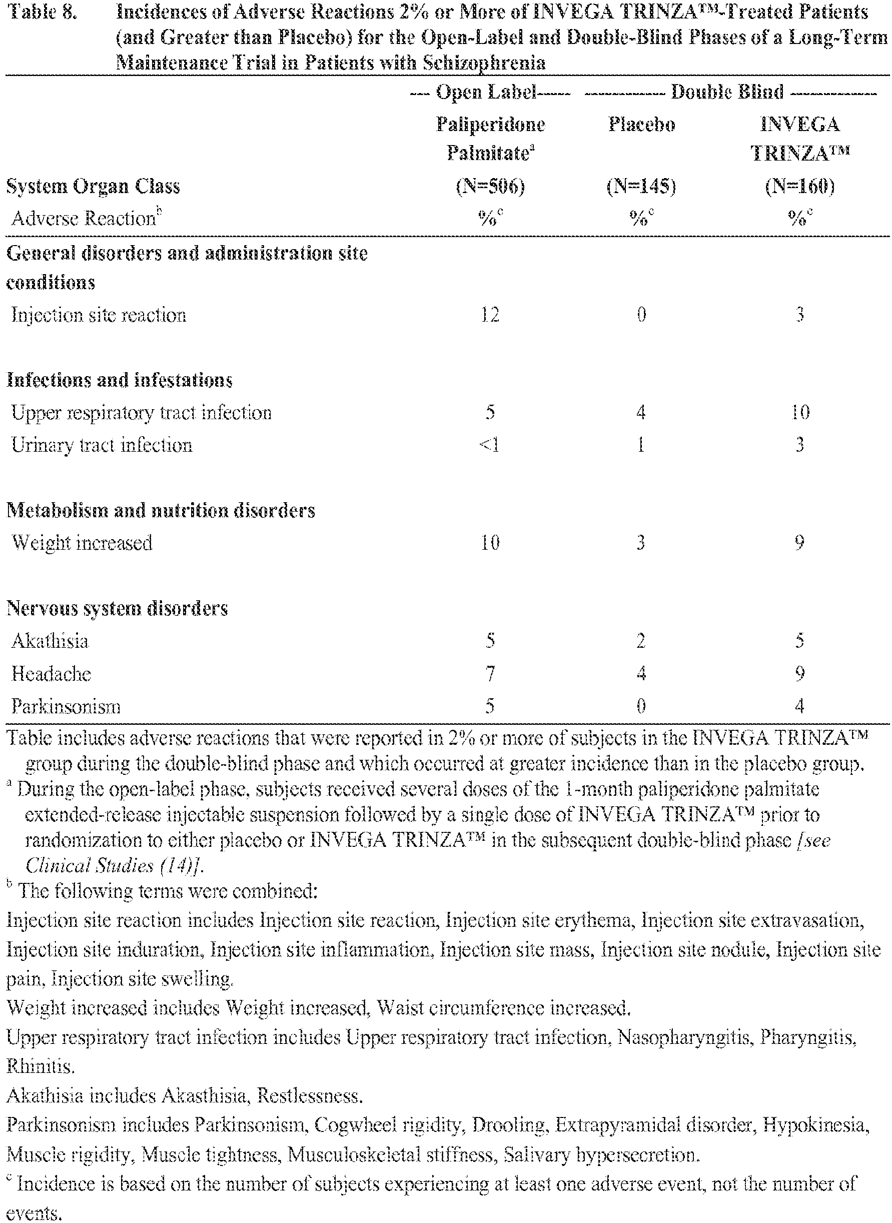

### Demographic Differences

An examination of population subgroups in the long-term maintenance trial did not reveal any evidence of differences in safety on the basis of age, gender, or race alone; however, there were few subjects 65 years of age and older.

### Extrapyramidal Symptoms (EPS)

Data from the long-term maintenance trial provided information regarding EPS. Several methods were used to measure EPS: (1) the Simpson-Angus global score which broadly evaluates parkinsonism, (2) the Barnes Akathisia Rating Scale global clinical rating score which evaluates akathisia, (3) the Abnormal Involuntary Movement Scale scores which evaluates dyskinesia, and (4) use of anticholinergic medications to treat EPS (Table 9), and (5) incidence of spontaneous reports of EPS (Table 10).

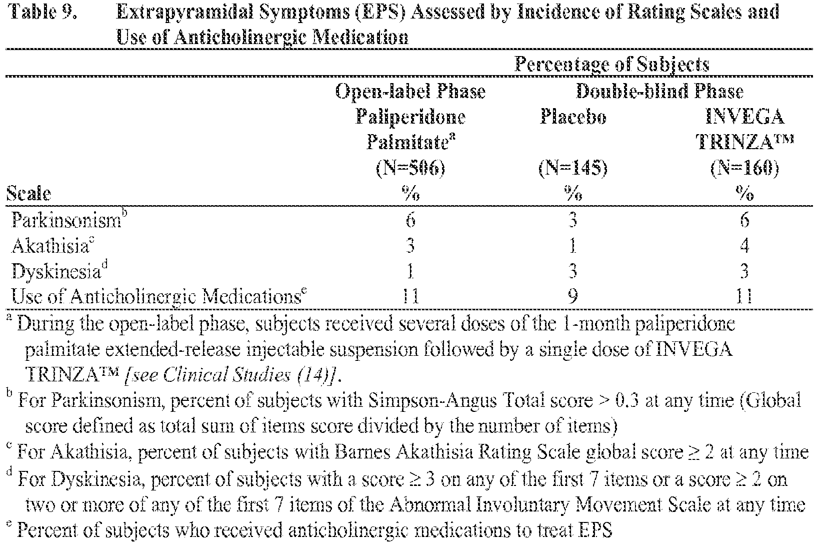

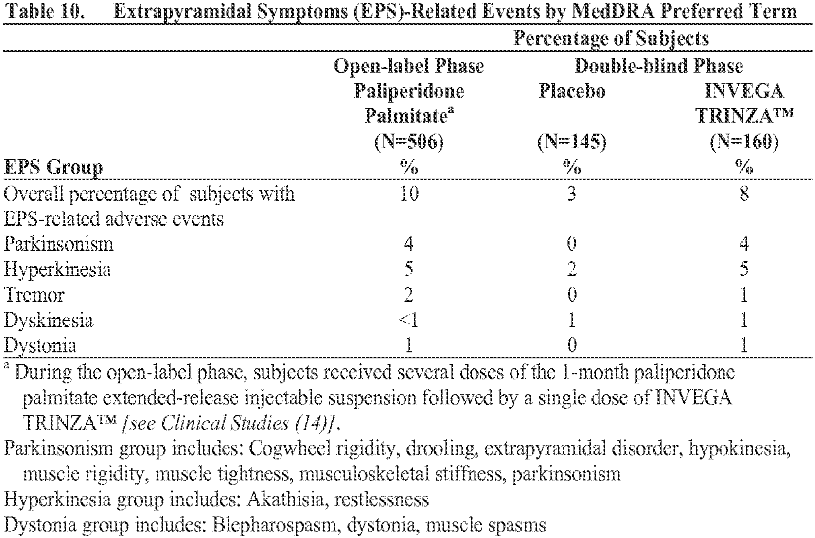

After injection of INVEGA TRINZA^{™} in the open-label phase, 12 (3.2%) subjects had EPS that were new or worsened in severity, with events under the groupings of hyperkinesia (1.6%) and parkinsonism (1.3%) being the most common. After injection of INVEGA TRINZA^{™} in the open-label or double-blind phases, one subject discontinued from the open-label phase due to restlessness.

An examination of the time to EPS during the double-blind phase showed no clustering of these events at visits that would be expected to correspond to median peak plasma concentrations of paliperidone for subjects randomized to INVEGA TRINZA^{™}.

### Dystonia

Symptoms of dystonia, prolonged abnormal contractions of muscle groups, may occur in susceptible individuals during the first few days of treatment. Dystonic symptoms include: spasm of the neck muscles, sometimes progressing to tightness of the throat, swallowing difficulty, difficulty breathing, and/or protrusion of the tongue. While these symptoms can occur at low doses, they occur more frequently and with greater severity with high potency and at higher doses of first generation antipsychotic drugs. An elevated risk of acute dystonia is observed in males and younger age groups.

### Pain Assessment and Local Injection Site Reactions

***Investigator ratings of injection site.*** Redness and swelling were observed in 2% or less of subjects in the INVEGA TRINZA^{™} and placebo groups during the double-blind phase of the long-term maintenance study, and were rated mild based on investigator ratings using a 4-point scale (0=absent; 1=mild; 2=moderate; 3=severe). There were no reports of induration in either group during the double-blind phase, and no subjects discontinued due to INVEGA TRINZA^{™} injection.

***Subject ratings of injection site pain.*** Subject evaluations of injection pain during the double-blind phase also were similar for placebo and INVEGA TRINZA^{™}.

Subject ratings of injection site pain in the single-dose Phase 1 study allowed for assessment of the temporal course of injection site pain. Residual injection pain peaked 1 or 6 hours after injection, and trended downward 3 days after the injection. Deltoid injections were numerically more painful than gluteal injections, although most pain ratings were below 10 mm on a 100-mm scale.

### Other Adverse Reactions Observed During the Clinical Trial Evaluation of INVEGA TRINZA^{™}

The following additional adverse reactions were identified in the long-term maintenance trial. The following list does not include reactions: 1) already listed in previous tables or elsewhere in labeling, 2) for which a drug cause was remote, 3) which were so general as to be uninformative, 4) which were not considered to have significant clinical implications, or 5) occurred at an incidence lower than that of placebo-treated patients.
**Cardiac disorders:** tachycardia
**Gastrointestinal disorders:** nausea, vomiting
**Metabolism and nutrition disorders:** hyperinsulinemia
**Psychiatric disorders:** anxiety

### Additional Adverse Reactions Reported in Clinical Trials with the 1-Month Paliperidone Palmitate Extended-Release Injectable Suspension

The following is a list of additional adverse reactions that have been reported in clinical trials with the 1-month paliperidone palmitate extended-release injectable suspension:
**Cardiac disorders:** atrioventricular block first degree, bradycardia, bundle branch block, palpitations, postural orthostatic tachycardia syndrome
**Ear and labyrinth disorders:** vertigo
**Eye disorders:** eye movement disorder, eye rolling, oculogyric crisis, vision blurred
**Gastrointestinal disorders:** abdominal discomfort/abdominal pain upper, diarrhea, dry mouth, toothache
**General disorders and administration site conditions:** asthenia, fatigue
**Immune system disorders:** hypersensitivity
**Investigations:** electrocardiogram abnormal
**Metabolism and nutrition disorders:** decreased appetite, increased appetite
**Musculoskeletal and connective tissue disorders:** back pain, myalgia, pain in extremity, joint stiffness, muscle spasms, muscle twitching, nuchal rigidity
**Nervous system disorders:** bradykinesia, cerebrovascular accident, convulsion, dizziness, dizziness postural, dysarthria, hypertonia, lethargy, oromandibular dystonia, psychomotor hyperactivity, syncope
**Psychiatric disorders:** agitation, nightmare
**Reproductive system and breast disorders:** breast discharge, erectile dysfunction, gynecomastia, menstrual disorder, menstruation delayed, menstruation irregular, sexual dysfunction
**Respiratory, thoracic and mediastinal disorders:** cough
**Skin and subcutaneous tissue disorders:** drug eruption, pruritus, pruritus generalized, rash, urticaria
**Vascular disorders:** hypertension

### Additional Adverse Reactions Reported in Clinical Trials with Oral Paliperidone

The following is a list of additional adverse reactions that have been reported in clinical trials with oral paliperidone:
**Cardiac disorders:** bundle branch block left, sinus arrhythmia
**Gastrointestinal disorders:** abdominal pain, constipation, flatulence, small intestinal obstruction
**General disorders and administration site conditions:** edema, edema peripheral
**Immune system disorders:** anaphylactic reaction
**Musculoskeletal and connective tissue disorders:** arthralgia, musculoskeletal pain, torticollis, trismus
**Nervous system disorders:** grand mal convulsion, parkinsonian gait, transient ischemic attack
**Psychiatric disorders:** sleep disorder
**Reproductive system and breast disorders:** breast engorgement, breast tenderness/breast pain, retrograde ejaculation
**Respiratory, thoracic and mediastinal disorders:** nasal congestion, pharyngolaryngeal pain, pneumonia aspiration
**Skin and subcutaneous tissue disorders:** rash papular
**Vascular disorders:** hypotension, ischemia

### 6.2 Postmarketing Experience

The following adverse reactions have been identified during postapproval use of paliperidone; because these reactions were reported voluntarily from a population of uncertain size, it is not always possible to reliably estimate their frequency or establish a causal relationship to drug exposure: angioedema, ileus, swollen tongue, thrombotic thrombocytopenic purpura, urinary incontinence, and urinary retention.

Cases of anaphylactic reaction after injection with the 1-month paliperidone palmitate extended-release suspension have been reported during postmarketing experience in patients who have previously tolerated oral risperidone or oral paliperidone.

Paliperidone is the major active metabolite of risperidone. Adverse reactions reported with oral risperidone and risperidone long-acting injection can be found in the *Adverse Reactions (6)* sections of the package inserts for those products.

### 7 DRUG INTERACTIONS

### 7.1 Drugs Having Clinically Important Interactions with INVEGA TRINZA^{™}

Because paliperidone palmitate is hydrolyzed to paliperidone *[see Clinical Pharmacology (12.3)]*, results from studies with oral paliperidone should be taken into consideration when assessing drug-drug interaction potential. In addition, consider the 3-month dosing interval and long half-life of INVEGA TRINZA *[see Dosage and Administration (2.1) and Clinical Pharmacology (12.3)].*

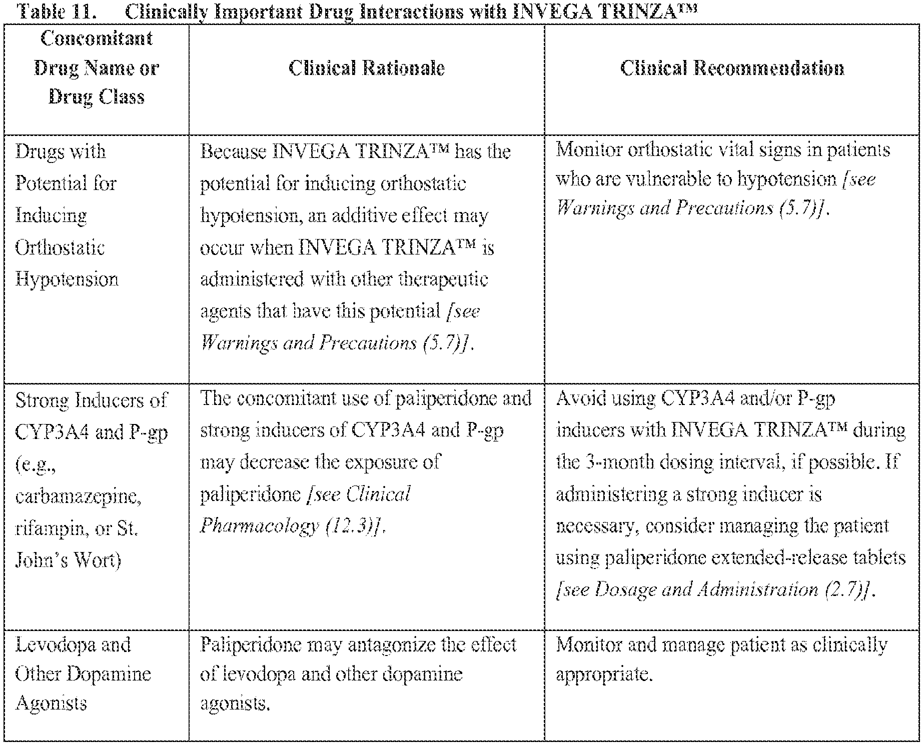

### 7.2 Drugs Having No Clinically Important Interactions with INVEGA TRINZA^{™}

Based on pharmacokinetic studies with oral paliperidone, no dosage adjustment of INVEGA TRINZA^{™} is required when administered concomitantly with valproate *[see Clinical Pharmacology (12.3)].* Additionally, no dosage adjustment is necessary for valproate when coadministered with INVEGA TRINZA^{™} *[see Clinical Phannacology (12.3)].*

Pharmacokinetic interaction between lithium and INVEGA TRINZA^{™} is unlikely.

Paliperidone is not expected to cause clinically important pharmacokinetic interactions with drugs that are metabolized by cytochrome P450 isozymes. *In vitro* studies indicate that CYP2D6 and CYP3A4 may be involved in paliperidone metabolism; however, there is no evidence *in vivo* that inhibitors of these enzymes significantly affect the metabolism of paliperidone. Paliperidone is not a substrate of CYP1A2, CYP2A6, CYP2C9, and CYP2C19; an interaction with inhibitors or inducers of these isozymes is unlikely. *[See Clinical Pharmacology (12.3)]*

### 8 USE IN SPECIFIC POPULATIONS

### 8.1 Pregnancy

### Pregnancy Exposure Registry

There is a pregnancy exposure registry that monitors pregnancy outcomes in women exposed to atypical antipsychotics, including INVEGA TRINZA, during pregnancy. For more information contact the National Pregnancy Registry for Atypical Antipsychotics at 1-866-961-2388 or visit *http:*//*womensmentalhealth.org*/*clinical-and-research-programs*/*pregnancyregistry*/*.*

### Risk Summary

Neonates exposed to antipsychotic drugs during the third trimester of pregnancy are at risk for extrapyramidal and/or withdrawal symptoms following delivery. There are no available data on INVEGA TRINZA^{™} use in pregnant women to inform any drug-associated risks for birth defects or miscarriage. Paliperidone has been detected in plasma at very low levels up to 18 months after a single-dose administration of INVEGA TRINZA^{™}, and the clinical significance of INVEGA TRINZA^{™} administered before pregnancy or anytime during pregnancy is not known *[see Clinical Pharmacology (12.3)].* No teratogenicity was observed when pregnant rats were injected intramuscularly with the 1-month paliperidone palmitate extended-release injectable suspension during organogenesis at doses up to 250 mg/kg, which is 3 times the maximum recommended human dose (MRHD) of 819 mg of the 3-month paliperidone palmitate injectable suspension on mg/m² basis.

Advise pregnant women of the potential risk to a fetus. The background risk of major birth defects and miscarriage for the indicated population are unknown. In the U.S. general population, the estimated background risk of major birth defects and miscarriage in clinically recognized pregnancies is 2-4% and 15-20%, respectively.

### Clinical Considerations

### Fetal/Neonatal Adverse Reactions

Extrapyramidal and/or withdrawal symptoms, including agitation, hypertonia, hypotonia, tremor, somnolence, respiratory distress, and feeding disorder have been reported in neonates who were exposed to antipsychotic drugs during the third trimester of pregnancy. These symptoms have varied in severity. Monitor neonates for extrapyramidal and/or withdrawal symptoms and manage symptoms appropriately. Some neonates recover within hours or days without specific treatment; others may require prolonged hospitalization.

### Data

### Human Data

There have been reports of agitation, hypertonia, hypotonia, tremor, somnolence, respiratory distress, and feeding disorder in neonates following *in utero* exposure to antipsychotics in the third trimester. These complications have varied in severity; while in some cases symptoms have been self-limited, in other cases neonates have required intensive care unit support and prolonged hospitalization.

### Animal Data

No developmental toxicity studies were conducted with the 3-month paliperidone palmitate extended-release injectable suspension.

No treatment-related effects on the offspring were observed when pregnant rats were injected intramuscularly with 1-month paliperidone palmitate extended-release injectable suspension during the period of organogenesis at doses up to 250 mg/kg, which is 3 times the MRHD of 819 mg of the 3-month paliperidone palmitate extended-release injectable suspension on mg/m² basis.

No increases in fetal abnormalities were observed when paliperidone was administered orally to pregnant rats and rabbits during the period of organogenesis at doses up to 8 times the MRHD of 12 mg/day of oral paliperidone on mg/m² basis.

In rat reproduction studies with risperidone, which is extensively converted to paliperidone in rats and humans, pup deaths increased at oral doses, which are less than the MRHD of risperidone on mg/m² basis (see RISPERDAL^{®} package insert).

### 8.2 Lactation

Paliperidone is present in human breast milk; however, there are insufficient data to assess the amount in human milk, the effects on the breastfed infant, or the effects on milk production. Paliperidone has been detected in plasma at very low levels up to 18 months after a single-dose administration of INVEGA TRINZA^{™}, and the clinical significance on the breastfed infant is not known *[see Clinical Pharmacology (12.3)].*

### 8.4 Pediatric Use

Safety and effectiveness of INVEGA TRINZA^{™} in patients less than 18 years of age have not been established. Use of INVEGA TRINZA^{™} is not recommended in pediatric patients because of the potential longer duration of an adverse event compared to shorter-acting products. In clinical trials of oral paliperidone, there were notably higher incidences of dystonia, hyperkinesia, tremor, and parkinsonism in the adolescent population as compared to the adult studies.

### Juvenile Animal Data

No juvenile animal studies were conducted with the 3-month paliperidone palmitate extended-release injectable suspension.

Juvenile rats administered daily oral doses of paliperidone from days 24 to 73 of age had a reversible impairment of performance in a test of learning and memory in females only. The no-effect dose of 0.63 mg/kg/day produced plasma exposure (AUC) to paliperidone similar to that in adolescents. No other consistent effects on neurobehavior or reproductive development were seen up to the highest dose tested which produced plasma exposure to paliperidone 2 to 3 times that in adolescents.

Juvenile dogs administered for 40 weeks daily oral doses of risperidone, which is extensively metabolized to paliperidone in animals and humans, at 0.31, 1.25, and 5 mg/kg/day, had decreased bone length and density with no-effect dose of 0.31 mg/kg/day, which produced plasma levels (AUC) of risperidone plus paliperidone similar to those in children and adolescents receiving the MRHD of 6 mg/day of risperidone. In addition, delayed sexual maturation was seen at all doses in both males and females. All adverse effects showed little or no reversibility in females after a 12-week drug-free recovery period.

The long-term effects of paliperidone on growth and sexual maturation have not been fully evaluated in children and adolescents.

### 8.5 Geriatric Use

Clinical studies of INVEGA TRINZA^{™} did not include sufficient numbers of subjects aged 65 and over to determine whether they respond differently from younger subjects. Other reported clinical experience has not identified differences in responses between the elderly and younger patients.

This drug is known to be substantially excreted by the kidney and clearance is decreased in patients with renal impairment *[see Clinical Pharmacology (12.3)],* who should be given reduced doses. Because elderly patients are more likely to have decreased renal function, monitor renal function and adjust dosage *[see Dosage and Administration (2.5)].*

### 8.6 Renal Impairment

Use of INVEGA TRINZA^{™} is not recommended in patients with moderate or severe renal impairment (creatinine clearance < 50 mL/min). Use of INVEGA TRINZA^{™} in patients with mild renal impairment (creatinine clearance ≥ 50 mL/min to < 80 mL/min) is based on the previous dose of the 1-month paliperidone palmitate extended-release injectable suspension that the patient was stabilized on prior to initiation of INVEGA TRINZA^{™} *[see Dosage and Administration (2.5) and Clinical Pharmacology (12.3)].*

### 8.7 Hepatic Impairment

INVEGA TRINZA^{™} has not been studied in patients with hepatic impairment. Based on a study with oral paliperidone, no dose adjustment is required in patients with mild or moderate hepatic impairment. Paliperidone has not been studied in patients with severe hepatic impairment *[see Clinical Pharmacology (12.3)].*

### 8.8 Patients with Parkinson's Disease or Lewy Body Dementia

Patients with Parkinson's Disease or Dementia with Lewy Bodies can experience increased sensitivity to INVEGA TRINZA^{™}. Manifestations can include confusion, obtundation, postural instability with frequent falls, extrapyramidal symptoms, and clinical features consistent with neuroleptic malignant syndrome.

### 9 DRUG ABUSE AND DEPENDENCE

### 9.1 Controlled Substance

INVEGA TRINZA^{™} (paliperidone) is not a controlled substance.

### 9.2 Abuse

Paliperidone has not been systematically studied in animals or humans for its potential for abuse.

### 9.3 Dependence

Paliperidone has not been systematically studied in animals or humans for its potential for tolerance or physical dependence.

### 10 OVERDOSAGE

### 10.1 Human Experience

No cases of overdose were reported in premarketing studies with paliperidone palmitate injection. Because INVEGA TRINZA^{™} is to be administered by health care professionals, the potential for overdosage by patients is low.

While experience with paliperidone overdose is limited, among the few cases of overdose reported in premarketing trials with oral paliperidone, the highest estimated ingestion was 405 mg. Observed signs and symptoms included extrapyramidal symptoms and gait unsteadiness. Other potential signs and symptoms include those resulting from an exaggeration of paliperidone's known pharmacological effects, i.e., drowsiness and sedation, tachycardia and hypotension, and QT prolongation. Torsades de pointes and ventricular fibrillation have been reported in a patient in the setting of overdose with oral paliperidone.

Paliperidone is the major active metabolite of risperidone. Overdose experience reported with risperidone can be found in the *OVERDOSAGE* section of the risperidone package insert.

### 10.2 Management of Overdosage

Contact a Certified Poison Control Center for the most up to date information on the management of paliperidone and INVEGA TRINZA^{™} overdosage (1-800-222-1222 or www.poison.org). Provide supportive care. including close medical supervision and monitoring. Treatment should consist of general measures employed in the management of overdosage with any drug. Consider the possibility of multiple drug overdosage. Ensure an adequate airway, oxygenation, and ventilation. Monitor cardiac rhythm and vital signs. Use supportive and symptomatic measures. There is no specific antidote to paliperidone.

Consider the prolonged-release characteristics of INVEGA TRINZA^{™} and the long apparent half-life of paliperidone when assessing treatment needs and recovery.

### 11 DESCRIPTION

INVEGA TRINZA^{™} is an atypical antipsychotic. INVEGA TRINZA^{™} contains paliperidone palmitate. The active ingredient, paliperidone palmitate, is a psychotropic agent belonging to the chemical class of benzisoxazole derivatives. INVEGA TRINZA^{™} contains a racemic mixture of (+)- and (-)- paliperidone palmitate. The chemical name is (9RS)-3-[2-[4-(6-Fluoro-1,2-benzisoxazol-3-yl)piperidin-1-yl]ethyl]-2-methyl-4-oxo-6,7,8,9-tetrahydro-4H-pyrido[1,2-a]pyrimadin-9-yl hexadecanoate. Its molecular formula is C₃₉H₅₇FN₄O₄ and its molecular weight is 664.89. The structural formula is:

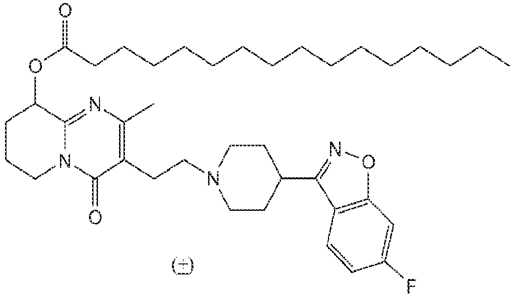

Paliperidone palmitate is very slightly soluble in ethanol and methanol, practically insoluble in polyethylene glycol 400 and propylene glycol, and slightly soluble in ethyl acetate.

INVEGA TRINZA^{™} is available as a white to off-white sterile aqueous extended-release suspension for intramuscular injection in dose strengths of 273 mg, 410 mg, 546 mg, and 819 mg paliperidone palmitate. The drug product hydrolyzes to the active moiety, paliperidone, resulting in dose strengths of 175 mg, 263 mg, 350 mg, and 525 mg of paliperidone, respectively. The inactive ingredients are polysorbate 20 (10 mg/mL), polyethylene glycol 4000 (75 mg/mL), citric acid monohydrate (7.5 mg/mL), sodium dihydrogen phosphate monohydrate, sodium hydroxide, and water for injection.

INVEGA TRINZA^{™} is provided in a prefilled syringe (cyclic-olefin-copolymer) prefilled with either 175 mg (0.875 mL), 263 mg (1.315 mL), 350 mg (1.75 mL), or 525 mg (2.625 mL) paliperidone (as 273 mg, 410 mg, 546 mg, or 819 mg paliperidone palmitate) suspension with a plunger stopper and tip cap (bromobutyl rubber), a backstop, and 2 types of commercially available needles: a thin walled 22G, 1 ½-inch safety needle and a thin walled 22G, 1-inch safety needle.

### 12 CLINICAL PHARMACOLOGY

### 12.1 Mechanism of Action

Paliperidone palmitate is hydrolyzed to paliperidone *[see Clinical Pharmacology (12.3)].* The mechanism of action of paliperidone is unknown. It has been proposed that the therapeutic activity of paliperidone in schizophrenia is mediated through a combination of central dopamine Type 2 (D₂) and serotonin Type 2 (5HT_{2A}) receptor antagonism.

### 12.2 Pharmacodynamics

Paliperidone is a centrally active dopamine Type 2 (D₂) receptor antagonist and a serotonin Type 2 (5HT_{2A}) receptor antagonist. Paliperidone is also active as an antagonist at α₁ and α₂ adrenergic receptors and H₁ histaminergic receptors, which may explain some of the other effects of the drug. Paliperidone has no affinity for cholinergic muscarinic or β₁- and β₂-adrenergic receptors. The pharmacological activity of the (+) and (-)- paliperidone enantiomers is qualitatively and quantitatively similar *in vitro.*

### 12.3 Pharmacokinetics

### Absorption and Distribution

Due to its extremely low water solubility, the 3-month formulation of paliperidone palmitate dissolves slowly after intramuscular injection before being hydrolyzed to paliperidone and absorbed into the systemic circulation. The release of the drug starts as early as day 1 and lasts for as long as 18 months.

Following a single intramuscular dose of INVEGA TRINZA^{™}, the plasma concentrations of paliperidone gradually rise to reach maximum plasma concentrations at a median Tₘₐₓ of 30-33 days. Following intramuscular injection of INVEGA TRINZA^{™} at doses of 273-819 mg in the deltoid muscle, on average, an 11-12% higher Cₘₐₓ was observed compared with injection in the gluteal muscle. The release profile and dosing regimen of INVEGA TRINZA^{™} results in sustained therapeutic concentrations over 3 months. The total and peak exposure of paliperidone following INVEGA TRINZA^{™} administration was dose-proportional over a 273-819 mg dose range. The mean steady-state peak:trough ratio for a INVEGA TRINZA^{™} dose was 1.6 following gluteal administration and 1.7 following deltoid administration. Following administration of INVEGA TRINZA^{™}, the apparent volume of distribution of paliperidone is 1960 L.

The plasma protein binding of racemic paliperidone is 74%.

Following administration of INVEGA TRINZA^{™}, the (+) and (-) enantiomers of paliperidone interconvert, reaching an AUC (+) to (-) ratio of approximately 1.7-1.8.

### Metabolism and Elimination

In a study with oral immediate-release ¹⁴C-paliperidone, one week following administration of a single oral dose of 1 mg immediate-release ¹⁴C-paliperidone, 59% of the dose was excreted unchanged into urine, indicating that paliperidone is not extensively metabolized in the liver. Approximately 80% of the administered radioactivity was recovered in urine and 11% in the feces. Four metabolic pathways have been identified *in vivo,* none of which accounted for more than 10% of the dose: dealkylation, hydroxylation, dehydrogenation, and benzisoxazole scission. Although *in vitro* studies suggested a role for CYP2D6 and CYP3A4 in the metabolism of paliperidone, there is no evidence *in vivo* that these isozymes play a significant role in the metabolism of paliperidone. Population pharmacokinetics analyses indicated no discernible difference on the apparent clearance of paliperidone after administration of oral paliperidone between extensive metabolizers and poor metabolizers of CYP2D6 substrates.

The median apparent half-life of paliperidone following INVEGA TRINZA^{™} administration over the dose range of 273-819 mg ranged from 84-95 days following deltoid injections and 118-139 days following gluteal injections. The concentration of paliperidone remaining in the circulation 18 months after dosing of 819 mg INVEGA TRINZA^{™} is stopped is estimated to be 3% (following deltoid injection) or 7% (following gluteal injection) of the average steady-state levels.

### Long-acting 3-month paliperidone palmitate injection versus other paliperidone formulations

INVEGA TRINZA^{™} is designed to deliver paliperidone over a 3-month period, while 1-month paliperidone palmitate injection is administered on a monthly basis. INVEGA TRINZA^{™}, when administered at doses that are 3.5-fold higher than the corresponding dose of 1-month paliperidone palmitate injection, results in paliperidone exposures similar to those obtained with corresponding monthly doses of 1-month paliperidone palmitate injection and corresponding once daily doses of paliperidone extended-release tablets. The exposure range for INVEGA TRINZA^{™} is encompassed within the exposure range for the approved dose strengths of paliperidone extended-release tablets.

The between-subject variability for paliperidone pharmacokinetics following delivery from INVEGA TRINZA^{™} was similar to the variability for paliperidone extended-release tablets. Because of the difference in median pharmacokinetic profiles among the three formulations, caution should be exercised when making a direct comparison of their pharmacokinetic properties.

### Drug Interaction Studies

No specific drug interaction studies have been performed with INVEGA TRINZA^{™}, The information below is obtained from studies with oral paliperidone.

Effects of other drugs on the exposures of INVEGA TRINZA^{™} are summarized in Figure 1. After oral administration of 20 mg/day of paroxetine (a potent CYP2D6 inhibitor), an increase in mean Cₘₐₓ and AUC values at steady-state was observed (see Figure 1). Higher doses of paroxetine have not been studied. The clinical relevance is unknown. After oral administration, a decrease in mean Cₘₐₓ and AUC values at steady state is expected when patients are treated with carbamazepine, a strong inducer of both CYP3A4 and P-gp *[see Drug Interactions (7.1)].* This decrease is caused, to a substantial degree, by a 35% increase in renal clearance of paliperidone.

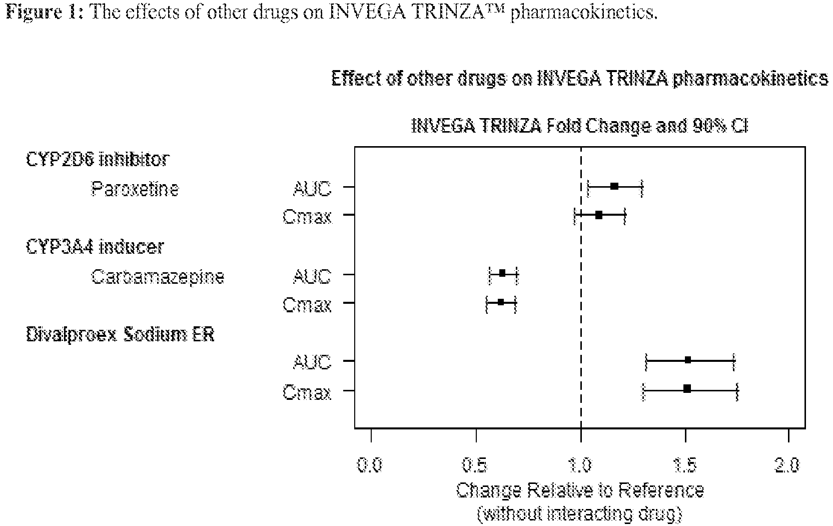

*In vitro* studies indicate that CYP2D6 and CYP3A4 may be involved in paliperidone metabolism, however, there is no evidence *in vivo* that inhibitors of these enzymes significantly affect the metabolism of paliperidone; they contribute to only a small fraction of total body clearance. *In vitro* studies demonstrated that paliperidone is a substrate of P-glycoprotein (P-gp) *[see Drug Interactions (7.2)].*

*In vitro* studies in human liver microsomes demonstrated that paliperidone does not substantially inhibit the metabolism of drugs metabolized by cytochrome P450 isozymes, including CYP1A2, CYP2A6, CYP2C8/9/10, CYP2D6, CYP2E1, CYP3A4, and CYP3A5. Therefore, paliperidone is not expected to inhibit clearance of drugs that are metabolized by these metabolic pathways in a clinically relevant manner. Paliperidone is also not expected to have enzyme inducing properties.

Paliperidone is a weak inhibitor of P-gp at high concentrations. No *in vivo* data are available, and the clinical relevance is unknown.

The effects of INVEGA TRINZA^{™} on the exposures of other drugs are summarized in Figure 2.

After oral administration of paliperidone, the steady-state Cₘₐₓ and AUC of valproate were not affected in 13 patients stabilized on valproate. In a clinical study, subjects on stable doses of valproate had comparable valproate average plasma concentrations when oral paliperidone extended-release tablets 3-15 mg/day was added to their existing valproate treatment *[see Drug Interactions (7.1)].*

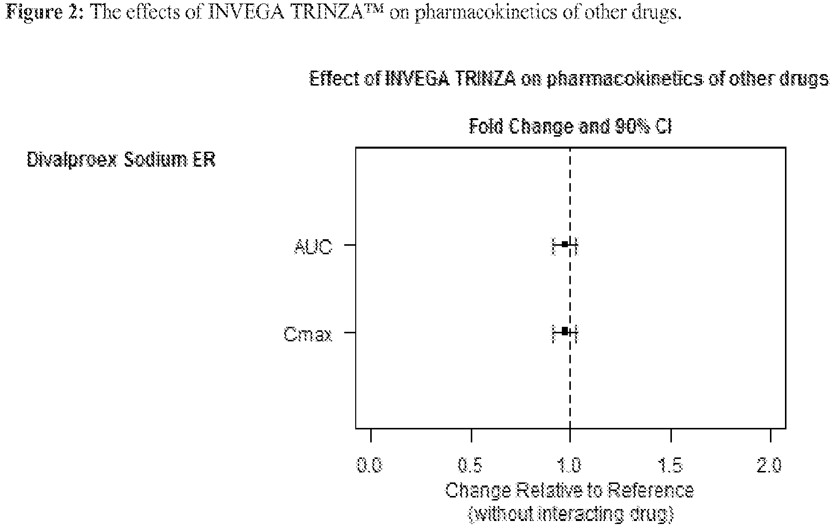

### Studies in Specific Populations

No specific pharmacokinetic studies have been performed with INVEGA TRINZA^{™} in specific populations. All the information is obtained from studies with oral paliperidone or is based on the population pharmacokinetic modelling of oral paliperidone and INVEGA TRINZA^{™}, Exposures of paliperidone in specific populations (renal impairment, hepatic impairment and elderly) are summarized in Figure 3 *[see Dosage and Administration (2.5) and Use in Specific Populations (8.6)].*

After oral administration of paliperidone in patients with moderate hepatic impairment, the plasma concentrations of free paliperidone were similar to those of healthy subjects, although total paliperidone exposure decreased because of a decrease in protein binding. Paliperidone has not been studied in patients with severe hepatic impairment *[see Use in Specific Populations (8.7)].*

After oral administration of paliperidone in elderly subjects, the Cₘₐₓ and AUC increased 1.2-fold compared to young subjects. However, there may be age-related decreases in creatinine clearance *[see Dosage and Administration (2.5) and Use in Specific Populations (8.5)].*

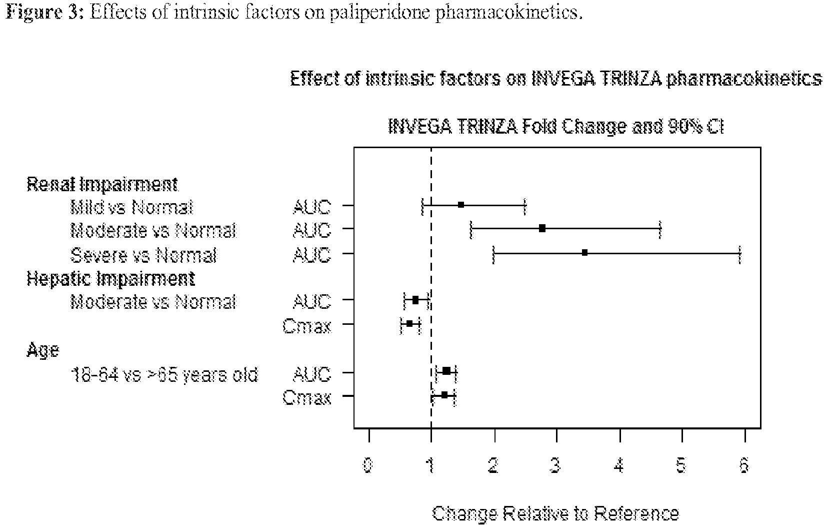

Based on *in vitro* studies utilizing human liver enzymes, paliperidone is not a substrate for CYP1A2; smoking should, therefore, not have an effect on the pharmacokinetics of paliperidone. Slower absorption was observed in females in a population pharmacokinetic analysis. At apparent steady-state with INVEGA TRINZA^{™}, the trough concentrations were similar between males and females.

Lower Cₘₐₓ was observed in overweight and obese subjects. At apparent steady-state with INVEGA TRINZA^{™}, the trough concentrations were similar among normal, overweight, and obese subjects.

### 13 NONCLINICAL TOXICOLOGY

### 13.1 Carcinogenesis, Mutagenesis, Impairment of Fertility

### Carcinogenesis

No carcinogenicity studies were conducted with the 3-month paliperidone palmitate extended-release injectable suspension.

The carcinogenic potential of intramuscular injection of 1-month paliperidone palmitate extended-release injectable suspension was assessed in rats. There was an increase in mammary gland adenocarcinomas in female rats at 16, 47, and 94 mg/kg/month, which are 0.2, 0.6 and 1 times the maximum recommended human dose (MRHD) of 819 mg of 3-month paliperidone palmitate extended-release injectable suspension on mg/m² basis. A no-effect dose was not established. Male rats showed an increase in mammary gland adenomas, fibroadenomas, and carcinomas at doses, which are 0.6 and 1 times the MRHD of 819 mg of 3-month paliperidone palmitate extended-release injectable suspension on mg/m² basis. A carcinogenicity study in mice has not been conducted with the 1-month paliperidone palmitate extended-release injectable suspension.

Carcinogenicity studies of risperidone, which is extensively converted to paliperidone in rats, mice, and humans, were conducted in Swiss albino mice and Wistar rats. Risperidone was administered in the diet for 18 months to mice and for 25 months to rats at daily doses of 0.63, 2.5, and 10 mg/kg/day, which are 0.2 to 3 times in mice and 0.4 to 6 times in rats the MRHD of 16 mg/day of risperidone on mg/m² basis. A maximum tolerated dose was not achieved in male mice. There were statistically significant increases in pituitary gland adenomas, endocrine pancreas adenomas, and mammary gland adenocarcinomas. The no-effect dose for these tumors was less than or equal to the MRHD of risperidone on mg/m² basis (see RISPERDAL^{®} package insert). An increase in mammary, pituitary, and endocrine pancreas neoplasms has been found in rodents after chronic administration of other antipsychotic drugs and is considered to be mediated by prolonged dopamine D2-receptor antagonism and hyperprolactinemia. The relevance of these tumor findings in rodents in terms of human risk is unknown *[see Warnings and Precautions (5.9)].*

### Mutagenesis

No mutagenesis studies were conducted with the 3-month paliperidone palmitate extended-release injectable suspension.

Paliperidone palmitate showed no genotoxicity in the *in vitro* Ames bacterial reverse mutation test and mouse lymphoma assay. Paliperidone was not genotoxic in the *in vitro* Ames bacterial reverse mutation test, mouse lymphoma assay and the *in vivo* rat bone marrow micronucleus test.

### Impairment of Fertility

No fertility studies were conducted with the 3-month paliperidone palmitate extended-release injectable suspension.

In a rat fertility study orally administered paliperidone increased pre- and post-implantation losses and slightly decreased the number of live embryos at doses up to 2.5 mg/kg/day, a dose which is 2 times the MRHD of 12 mg on mg/m² basis. This dose also caused slight maternal toxicity but there was no effect on the percentage of treated female rats that became pregnant. Pre- and post- implantation losses, the number of live embryos and maternal toxicity were not affected at 0.63 mg/kg/day, a dose, which is half of the MRHD of 12 mg/day of orally administered paliperidone on mg/m² basis. The fertility of male rats was not affected at oral doses of paliperidone of up to 2.5 mg/kg/day, which are up to 2 times the MRHD of 12 mg on mg/m² basis, although sperm count and sperm viability studies were not conducted with paliperidone.

In a sub-chronic study in Beagle dogs with risperidone, which is extensively converted to paliperidone in dogs and humans, all doses tested 0.31 to 5.0 mg/kg/day, which are 0.6 to 10 times the MRHD of 16 mg on mg/m² basis, resulted in decreases in serum testosterone and decreases in sperm motility and concentration. Serum testosterone and sperm parameters partially recovered, but remained decreased at the last observation two months after treatment was discontinued.

### 13.2 Animal Toxicology and/or Pharmacology

Injection site toxicity was assessed in minipigs injected intramuscularly with the 3-month paliperidone palmitate extended-release injectable suspension at doses up to 819 mg, which is equal to the MRHD. Injection site inflammatory reactions were greater and more advanced than reactions to the 1-month paliperidone palmitate extended-release injectable suspension. Reversibility of these findings was not examined.

### 14 CLINICAL STUDIES

The efficacy of INVEGA TRINZA^{™} for the treatment of schizophrenia in patients who have been adequately treated for at least 4 months with INVEGA SUSTENNA^{®} (1-month paliperidone palmitate extended-release injectable suspension) was evaluated in a long-term double-blind, placebo-controlled randomized-withdrawal trial designed to evaluate time to relapse involving adult subjects who met DSM-IV-TR criteria for schizophrenia.

Patients could enter the study with acute symptoms (if previously treated with oral antipsychotics) or be clinically stable (if treated with long-acting injectable antipsychotics [LAI]). All patients who previously received oral antipsychotics received the paliperidone palmitate -month initiation regimen (deltoid injections of 234 mg and 156 mg one week apart), while those patients switching from LAI medication were treated with the 1-month paliperidone palmitate extended-release injectable suspension in place of the next scheduled injection. Specifically:
- For patients entering the study who were already being treated with the 1-month paliperidone palmitate extended-release injectable suspension, their dosing remained unchanged. Patients who were currently receiving the 39 mg dose of 1-month paliperidone palmitate were not eligible to enroll in the study.
- Patients entering the study who were being treated with 25 mg, 37.5 mg, or 50 mg of RISPERDAL CONSTA^{®} (risperidone long-acting injection) were switched to 78 mg, 117 mg, or 156 mg, respectively, of the 1-month paliperidone palmitate administered in the deltoid muscle.
- Patients entering the study who were being treated with any other LAI product were switched to 234 mg of the 1-month paliperidone palmitate administered in the deltoid muscle.

This study consisted of the following three treatment periods:
- A 17-week flexible-dose open-label period with the 1-month paliperidone palmitate (first part of a 29-week open-label stabilization phase). A total of 506 patients entered this phase of the study. Dosing of the 1-month paliperidone palmitate was individualized based on symptom response, tolerability, and previous medication history. Specifically, the dose could be adjusted at the week 5 and 9 injections and the injection site could be deltoid or gluteal. The week 13 dose had to be the same as the week 9 dose. Patients had to be clinically stable at the end of this period before receiving INVEGA TRINZA^{™} at the week 17 visit. Clinical stability was defined as achieving a PANSS total score <70 at week 17. The PANSS is a 30-item scale that measures positive symptoms of schizophrenia (7 items), negative symptoms of schizophrenia (7 items), and general psychopathology (16 items), each rated on a scale of 1 (absent) to 7 (extreme); total PANSS scores range from 30 to 210.
- A 12-week open-label treatment period with INVEGA TRINZA^{™} (second part of a 29-week open-label stabilization phase). A total of 379 patients received a single-dose of INVEGA TRINZA^{™} which was a 3.5 multiple of the last dose of the 1-month paliperidone palmitate. Patients had to remain clinically stable before entry into the next period (double-blind). Clinical stability was defined as achieving a PANSS total score <70 and scores of ≤ 4 for seven specific PANSS items.
- A variable length double-blind treatment period. In this period, 305 stabilized patients were randomized 1:1 to continue treatment with INVEGA TRINZA^{™} or placebo until relapse, early withdrawal, or the end of study. Patients were randomized to the same dose of INVEGA TRINZA^{™} they received during the open-label phase (i.e., 273 mg, 410 mg, 546 mg, or 819 mg) or to placebo administered every 12 weeks. The numbers (%) of patients entering double-blind on each of the dose levels were 6 (4%) for 273 mg, 15 (9%) for 410 mg, 78 (49%) for 546 mg, and 61 (38%) for 819 mg.

The primary efficacy variable was time to first relapse. Relapse was pre-defined as emergence of one or more of the following: psychiatric hospitalization, ≥ 25% increase (if the baseline score was ≥ 40) or a to-point increase (if the baseline score was ≤ 40) in total PANSS score on two consecutive assessments, deliberate self-injury, violent behavior, suicidal/homicidal ideation, or a score of ≥ 5 (if the maximum baseline score was ≤ 3) or ≥ 6 (if the maximum baseline score was 4) on two consecutive assessments of the specific PANSS items.

A pre-planned interim analysis showed a statistically significantly longer time to relapse in patients treated with INVEGA TRINZA^{™} compared to placebo, and the study was stopped early because efficacy was demonstrated. The most common reason for relapse observed across both treatment groups was increase in the PANSS total score value, followed by psychiatric hospitalization.

Twenty-three percent (23%) of patients in the placebo group and 7.4% of patients in the INVEGA TRINZA^{™} group experienced a relapse event. The time to relapse was statistically significantly longer in patients randomized to the INVEGA TRINZA^{™} group than compared to placebo-treated patients. A Kaplan-Meier plot of time to relapse by treatment group is shown in Figure 4.

An examination of population subgroups did not reveal any clinically significant differences in responsiveness on the basis of gender, age, or race.

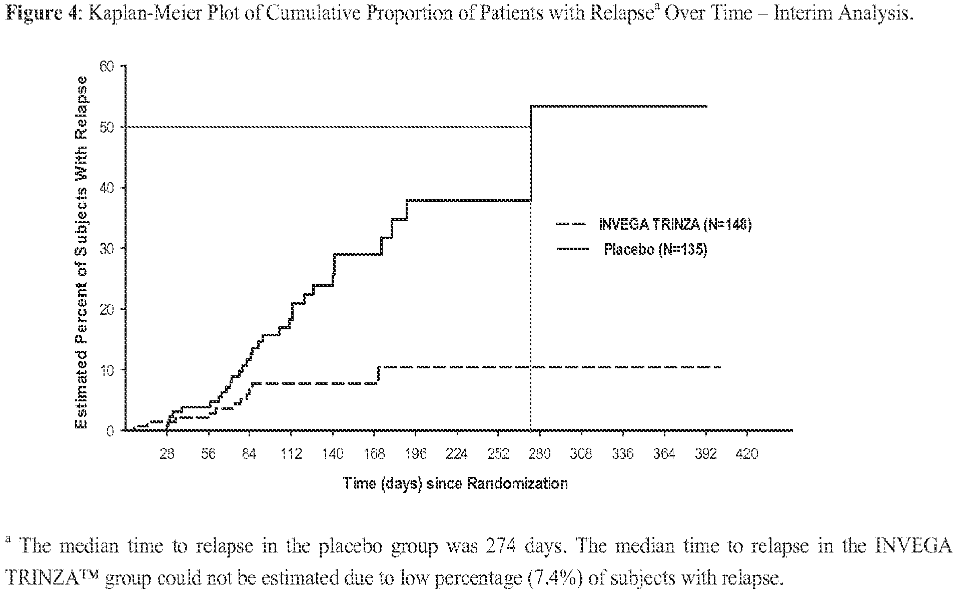

### 16 HOW SUPPLIED/STORAGE AND HANDLING

INVEGA TRINZA^{™} is available as a white to off-white sterile aqueous extended-release suspension for intramuscular injection in dose strengths of 273 mg, 410 mg, 546 mg, and 819 mg paliperidone palmitate. The kit contains a prefilled syringe and 2 safety needles (a thin walled 22G, 1-inch safety needle and a thin walled 22G, 1½-inch safety needle).
273 mg paliperidone palmitate kit (NDC 50458-606-01)
410 mg paliperidone palmitate kit (NDC 50458-607-01)
546 mg paliperidone palmitate kit (NDC 50458-608-01)
819 mg paliperidone palmitate kit (NDC 50458-609-01)

### Storage and Handling

Store at room temperature 20°C to 25°C (68°F to 77°F); excursions between 15°C and 30°C (59°F and 86°F) are permitted.

### 17 PATIENT COUNSELING INFORMATION

Advise the patient to read the FDA-approved patient labeling (Patient Information).

### Neuroleptic Malignant Syndrome (NMS)

Counsel patients about a potentially fatal side effect referred to as Neuroleptic Malignant Syndrome (NMS) that has been reported in association with administration of antipsychotic drugs. Patients should contact their health care provider or report to the emergency room if they experience the following signs and symptoms of NMS, including hyperpyrexia, muscle rigidity, altered mental status, and evidence of autonomic instability (irregular pulse or blood pressure, tachycardia, diaphoresis, and cardiac dysrhythmia *[see Warnings and Precautions (5.3)].*

### Tardive Dyskinesia

Counsel patients on the signs and symptoms of tardive dyskinesia and to contact their health care provider if these abnormal movements occur *[see Warnings and Precautions (5.5)].*

### Metabolic Changes

Educate patients about the risk of metabolic changes, how to recognize symptoms of hyperglycemia (high blood sugar) and diabetes mellitus (e.g., polydipsia, polyuria, polyphagia, and weakness), and the need for specific monitoring, including blood glucose, lipids, and weight *[see Warnings and Precautions (5.6)].*

### Orthostatic Hypotension

Educate patients about the risk of orthostatic hypotension, particularly at the time of initiating treatment, re-initiating treatment, or increasing the dose *[see Warnings and Precautions (5.7)].*

### Leukopenia/Neutropenia

Advise patients with a pre-existing low WBC or a history of drug induced leukopenia/neutropenia they should have their CBC monitored while taking INVEGA TRINZA^{™} *[see Warnings and Precautions (5.8)].*

### Hyperprolactinemia

Counsel patients on signs and symptoms of hyperprolactinemia that may be associated with chronic use of INVEGA TRINZA^{™}. Advise them to seek medical attention if they experience any of the following: amenorrhea or galactorrhea in females, erectile dysfunction or gynecomastia in males. *[See Warnings and Precautions (5.9)]*

### Interference with Cognitive and Motor Performance

As INVEGA TRINZA^{™} has the potential to impair judgment. thinking, or motor skills, caution patients about operating hazardous machinery, including automobiles, until they are reasonably certain that INVEGA TRINZA^{™} therapy does not affect them adversely *[see Warnings and Precautions (5.10)].*

### Priapism

Advise patients of the possibility of painful or prolonged penile erections (priapism). Instruct the patient to seek immediate medical attention in the event of priapism *[Warnings and Precautions (5.12)].*

### Heat Exposure and Dehydration

Counsel patients on the importance of avoiding overheating and dehydration *[see Warnings and Precautions (5.14)].*

### Concomitant Medication

Advise patients to inform their health care providers if they are taking, or plan to take any prescription or over-the-counter drugs, as there is a potential for interactions *[see Drug Interactions (7)].*

### Pregnancy

Advise patients that INVEGA TRINZA^{™} may cause extrapyramidal and/or withdrawal symptoms in a neonate and to notify their healthcare provider if they become pregnant or intend to become pregnant during treatment with INVEGA TRINZA^{™}. Advise patients that there is a pregnancy registry that monitors pregnancy outcomes in women exposed to INVEGA TRINZA during pregnancy *[see Use in Specific Populations (8.1)].*
INVEGA TRINZA^{™} (paliperidone palmitate) Extended-Release Injectable Suspension
INVEGA SUSTENNA^{®}, RISPERDAL^{®}, and RISPERDAL CONSTA^{®} are trademarks of Janssen Pharmaceuticals, Inc.

### Product of Ireland

Manufactured by:
Janssen Pharmaceutica N.V.
Beerse, Belgium

Manufactured for:
Janssen Pharmaceuticals, Inc.
Titusville, NJ 08560
^{©} Janssen Pharmaceuticals, Inc. 2015

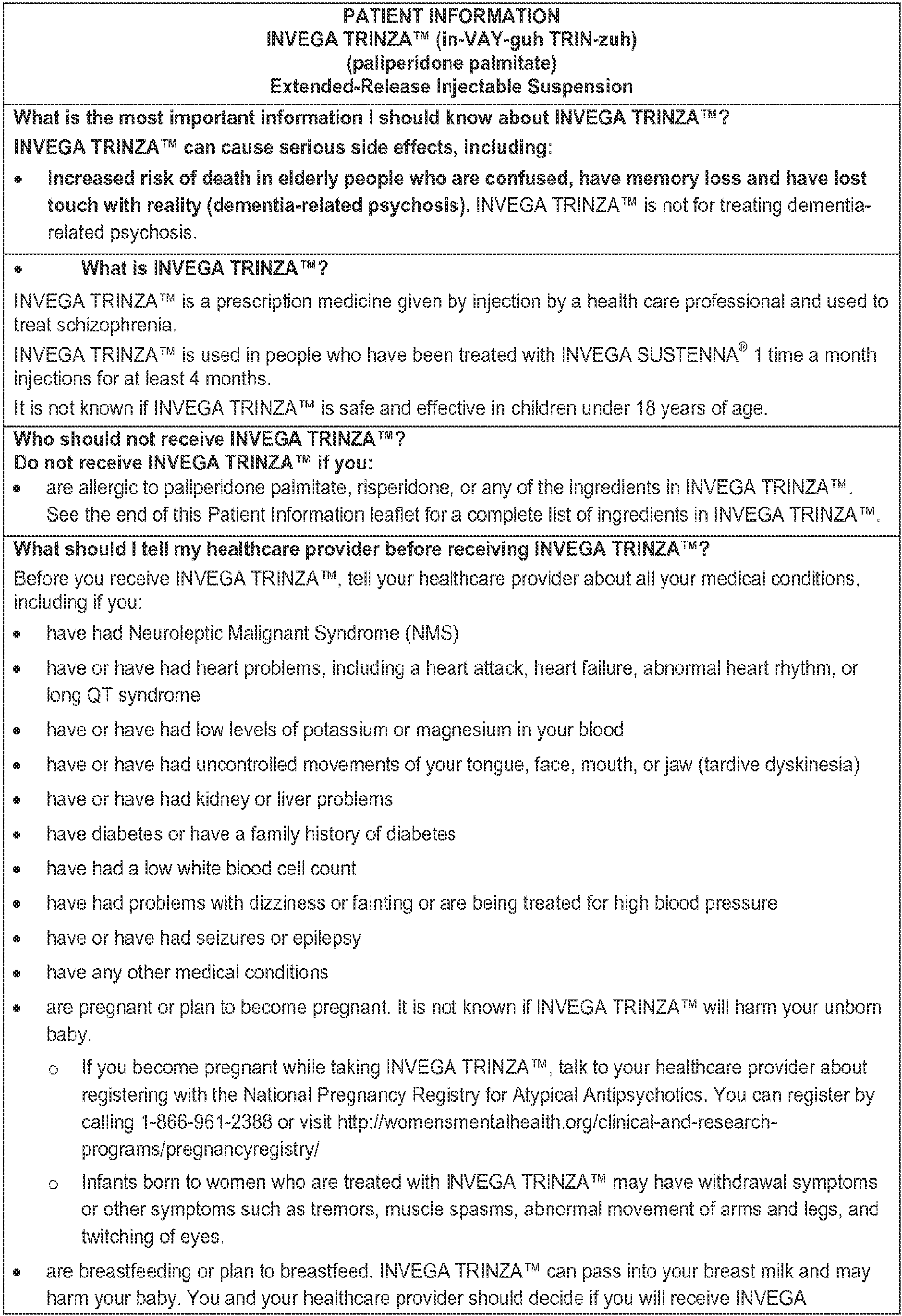

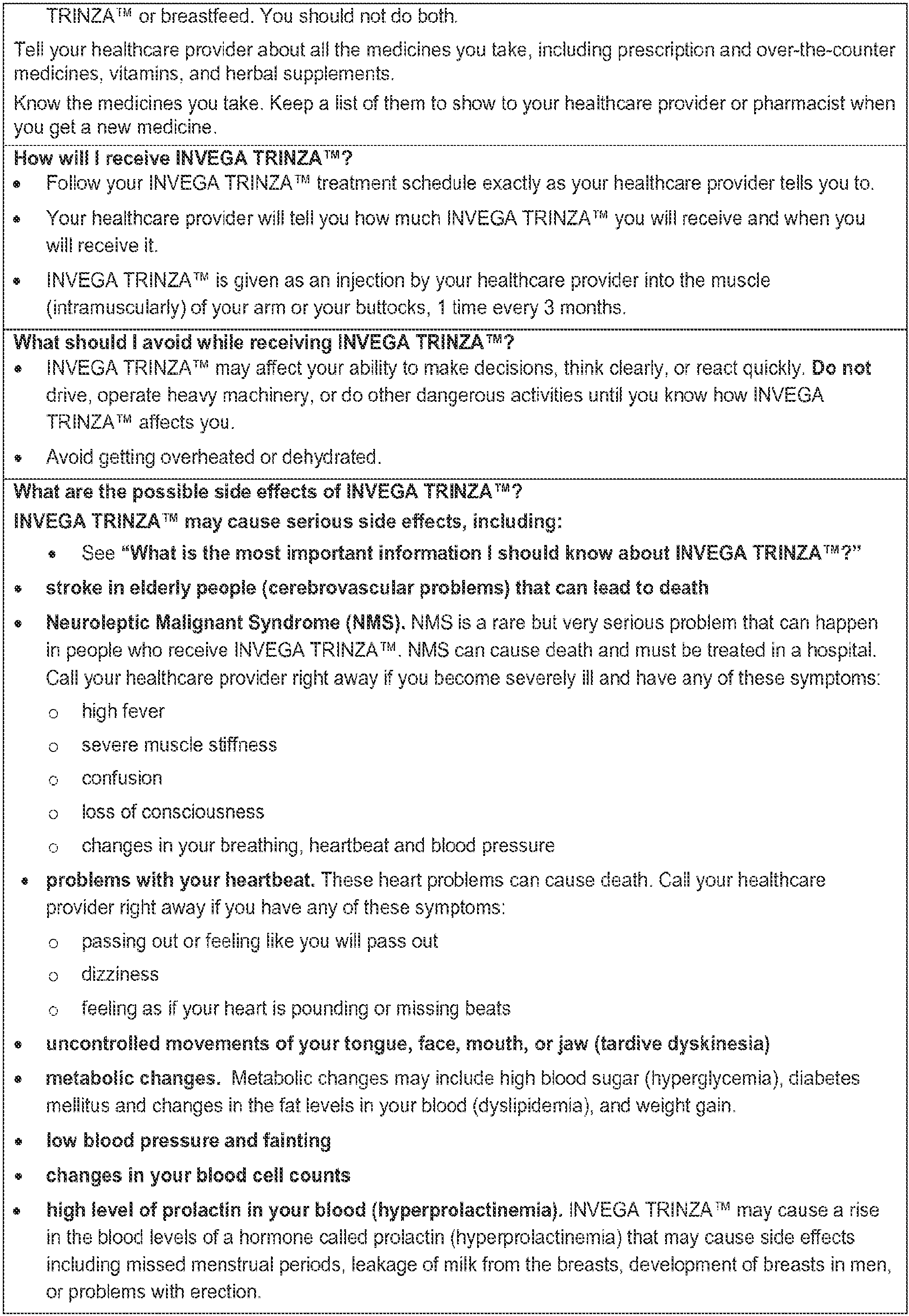

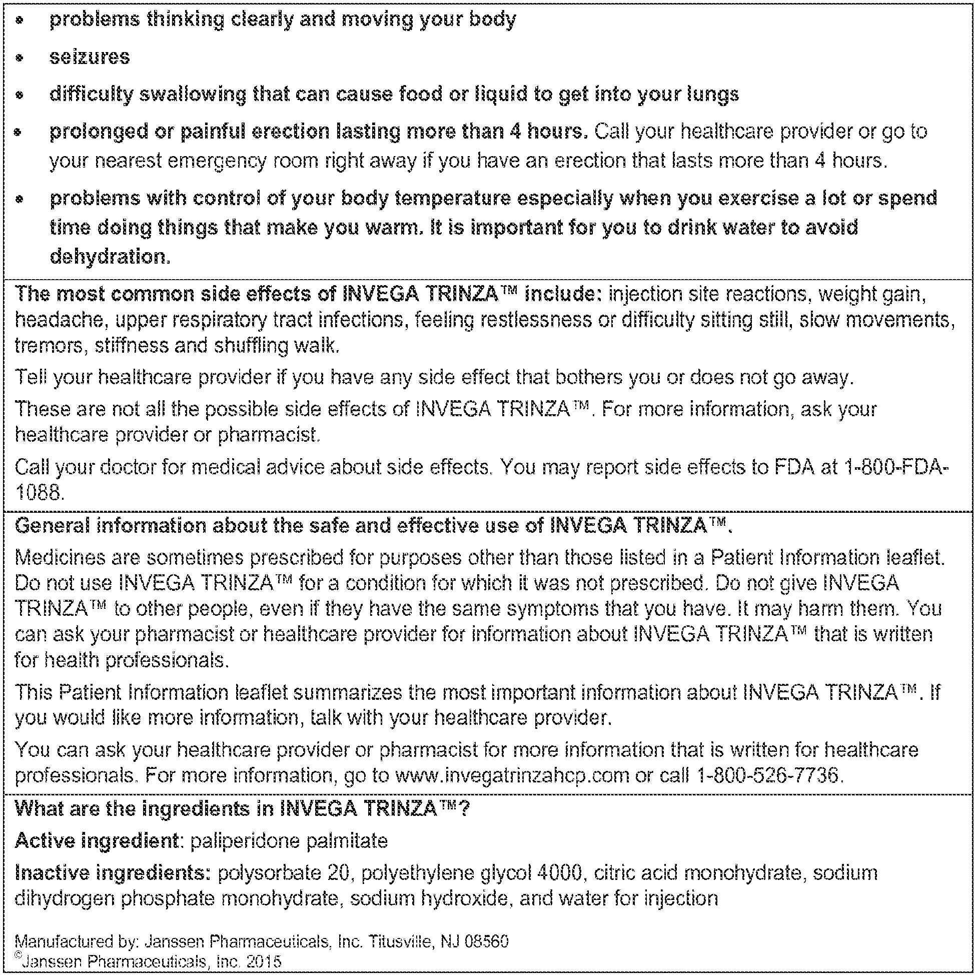

## Claims

1. A device for training users in a proper mixing of pharmaceutical components, the device comprising:
a housing that extends along a
longitudinal axis;
a power source (151) disposed in the
housing;
a microcontroller (154) disposed in the housing and electrically powered by the power source (151);
a user notification device (155); and
an accelerometer (150) disposed in the housing and electrically connected to the microcontroller (154) so that the microcontroller (154) is configured to detect a motion and orientation of the housing and indicate via the user notification device (155) as to whether the motion or orientation of the housing being shaken during one of a drug administration or a training event is sufficient with respect to predetermined thresholds including magnitude of the force applied during the shaking, the orientation of the housing and duration of such shaking, and wherein
the microcontroller (154) is configured to detect if the level of shaking vigor has reduced to a level below the pre-set threshold, and to enter a pause mode to allow the user to restart shaking during one of a drug administration or a training event.

2. A method to direct a user on a proper drug mixing technique with a training device or a device for mixing and assisting with the administration of the drug, that includes:
a housing that extends along a longitudinal axis;
a power source (151) disposed in the housing;
a microcontroller (154) disposed in the housing and electrically powered by the power source (151);
a user notification device (155); and
an accelerometer (150) disposed in the housing and electrically connected to the microcontroller (154) to detect motion and orientation of such motion,
the method comprising the steps of:
determining from the accelerometer (150) if the magnitude of the motion and orientation of the housing are sufficient with respect to predetermined thresholds including magnitude of the force applied during the shaking, the orientation of the housing and duration of such shaking; and
announcing via the user notification device (155) as to whether the motion or orientation of the housing being shaken during one of a drug administration or a training event meets the predetermined thresholds, and wherein
the microcontroller (154) is configured to detect if the level of shaking vigor has reduced to a level below the pre-set threshold, and to enter a pause mode to allow the user to restart the shaking during one of a drug administration or a training event.

3. The device of claim 1 or the method of claim 2, wherein the microcontroller (154) is configured to detect when shaking of the housing has ended prematurely and to enter a pause mode to allow the user to restart the shaking during one of a drug administration or a training event.

4. The device of claim 1 or the method of claim 2, wherein the device further includes a start switch (153) electrically connected to the microcontroller (154).

5. The device of claim 1 or the method of claim 2, in which the accelerometer (150) comprises a 3-axis accelerometer.

6. The device of claim 1 or the method of claim 2, in which the accelerometer (150) is configured to activate the microcontroller (154) upon detection of movements of the housing during one of a drug administration or a training event.

7. The device of claim 1 or the method of claim 2, in which the microcontroller (154) is configured to set a timer and determine when a maximum allowable time after shaking of the housing has elapsed to warn the user to shake the device again during one of a drug administration or a training event.

8. The device of claim 1 or the method of claim 2, in which the housing comprises a syringe barrel element (103) with finger flange (100) and one end and a barrel tip (104) spaced apart along the longitudinal axis.

9. The device of claim 1 or the method of claim 2, in which the housing comprises a body (109) with a slot (111) sized to accept a syringe barrel (107) or a vial.

10. The device of claim 1 or the method of claim 2, in which the housing comprises a housing provided with a compartment and a lid to receive an entire syringe.

11. The device of claim 1 or the method of claim 2, in which the housing comprises an elongated body approximately the same length as a syringe barrel such that a syringe is inserted into a syringe receiving hole in the body and retained by the compressive force applied by the finger-like members between a syringe finger flange and a body base.

12. The device of claim 1 or the method of claim 2, in which the housing comprises a body (127) with a syringe accepting slot (128) sized to accept a syringe barrel.

13. The device of claim 1 or the method of claim 2, in which the housing comprises a puck-like body with a syringe accepting hole so that in use a syringe is inserted into the syringe accepting hole and is held in place with a user's thumb on an underside thumb grip.

## Patentansprüche

1. Vorrichtung zum Trainieren von Benutzern in einem richtigen Mischen pharmazeutischer Komponenten, die Vorrichtung umfassend:
ein Gehäuse, das sich entlang einer Längsachse erstreckt;
eine Stromquelle (151), die in dem Gehäuse angeordnet ist;
eine Mikrosteuereinrichtung (154), die in dem Gehäuse angeordnet ist und durch die Stromquelle (151) mit Strom versorgt wird;
eine Benutzerbenachrichtigungsvorrichtung (155); und
einen Beschleunigungsmesser (150), der in dem Gehäuse angeordnet und mit der Mikrosteuereinrichtung (154) elektrisch verbunden ist, so dass die Mikrosteuereinrichtung (154) konfiguriert ist, um eine Bewegung und eine Ausrichtung des Gehäuses zu erkennen und über die Benutzerbenachrichtigungsvorrichtung (155) anzuzeigen, ob die Bewegung oder die Ausrichtung des Gehäuses, das während einem von einer Arzneimittelverabreichung oder einem Trainingsereignis geschüttelt wird, hinsichtlich zuvor bestimmter Schwellenwerte, einschließlich ein Ausmaß der Kraft, die während des Schüttelns aufgebracht wird, der Ausrichtung des Gehäuses und der Dauer eines derartigen Schüttelns, ausreichend ist, und wobei
die Mikrosteuereinrichtung (154) konfiguriert ist, um zu erkennen, falls der Pegel einer Schüttelintensität auf einen Pegel unterhalb des voreingestellten Schwellenwerts abgefallen ist, und um in einen Pausenmodus zu wechseln, um dem Benutzer zu ermöglichen, das Schütteln während einem von einer Arzneimittelverabreichung oder einem Trainingsereignis erneut zu starten.

2. Verfahren, um einem Benutzer eine richtige Technik zum Mischen von Arzneimitteln mit einer Trainingsvorrichtung oder einer Vorrichtung zum Mischen und Unterstützen der Verabreichung des Arzneimittels beizubringen, das einschließt:
ein Gehäuse, das sich entlang einer Längsachse erstreckt;
eine Stromquelle (151), die in dem Gehäuse angeordnet ist;
eine Mikrosteuereinrichtung (154), die in dem Gehäuse angeordnet ist und durch die Stromquelle (151) mit Strom versorgt wird;
eine Benutzerbenachrichtigungsvorrichtung (155); und
einen Beschleunigungsmesser (150), der in dem Gehäuse angeordnet und mit der Mikrosteuereinrichtung (154) elektrisch verbunden ist, um die Bewegung und die Ausrichtung einer derartigen Bewegung zu erkennen,
das Verfahren umfassend die folgenden Schritte:
Bestimmen von dem Beschleunigungsmesser (150), ob das Ausmaß der Bewegung und der Ausrichtung des Gehäuses hinsichtlich der zuvor bestimmten Schwellenwerte, einschließlich des Ausmaßes der Kraft, die während des Schüttelns aufgebracht wird, der Ausrichtung des Gehäuses und der Dauer eines derartigen Schüttelns, ausreichend sind; und
Bekanntgeben über die Benutzerbenachrichtigungsvorrichtung (155), ob die Bewegung oder die Ausrichtung des Gehäuses, das während einem von einer Arzneimittelverabreichung oder einem Trainingsereignis geschüttelt wird, die zuvor bestimmten Schwellenwerte erreicht, und wobei
die Mikrosteuereinrichtung (154) konfiguriert ist, um zu erkennen, falls der Pegel der Schüttelintensität unter den voreingestellten Schwellenwert abgefallen ist, und um in einen Pausenmodus zu wechseln, um dem Benutzer zu ermöglichen, das Schütteln während einem von einer Arzneimittelverabreichung oder einem Trainingsereignis erneut zu starten.

3. Vorrichtung nach Anspruch 1 oder Verfahren nach Anspruch 2, wobei die Mikrosteuereinrichtung (154) konfiguriert ist, um zu erkennen, wenn das Schütteln des Gehäuses vorzeitig beendet wurde, und um in einen Pausenmodus zu wechseln, um dem Benutzer zu ermöglichen, das Schütteln während einem von einer Arzneimittelverabreichung oder einem Trainingsereignis erneut zu starten.

4. Vorrichtung nach Anspruch 1 oder Verfahren nach Anspruch 2, wobei die Vorrichtung ferner einen Startschalter (153) einschließt, der mit der Mikrosteuereinrichtung (154) elektrisch verbunden ist.

5. Vorrichtung nach Anspruch 1 oder Verfahren nach Anspruch 2, bei der/dem der Beschleunigungsmesser (150) einen 3-Achsen-Beschleunigungsmesser umfasst.

6. Vorrichtung nach Anspruch 1 oder Verfahren nach Anspruch 2, bei der/dem der Beschleunigungsmesser (150) konfiguriert ist, um die Mikrosteuereinrichtung (154) bei dem Erkennen von Bewegungen des Gehäuses während einem von einer Arzneimittelverabreichung oder einem Trainingsereignis zu aktivieren.

7. Vorrichtung nach Anspruch 1 oder Verfahren nach Anspruch 2, bei der/dem die Mikrosteuereinrichtung (154) konfiguriert ist, um einen Zeitgeber einzustellen und zu bestimmen, wann eine maximal zulässige Zeit nach dem Schütteln des Gehäuses verstrichen ist, um den Benutzer zu warnen, die Vorrichtung während einem von einer Arzneimittelverabreichung oder einem Trainingsereignis erneut zu schütteln.

8. Vorrichtung nach Anspruch 1 oder Verfahren nach Anspruch 2, bei der/dem das Gehäuse ein Spritzenzylinderelement (103) mit Fingerflansch (100) und einem Ende und einer Zylinderspitze (104) umfasst, die entlang der Längsachse beabstandet sind.

9. Vorrichtung nach Anspruch 1 oder Verfahren nach Anspruch 2, bei der/dem das Gehäuse einen Körper (109) mit einem Schlitz (111) umfasst, der bemessen ist, um einen Spritzenzylinder (107) oder eine Phiole aufzunehmen.

10. Vorrichtung nach Anspruch 1 oder Verfahren nach Anspruch 2, bei der/dem das Gehäuse ein Gehäuse umfasst, das mit einem Fach und einem Deckel versehen ist, um eine ganze Spritze zu empfangen.

11. Vorrichtung nach Anspruch 1 oder Verfahren nach Anspruch 2, bei der/dem das Gehäuse einen länglichen Körper umfasst, der etwa die gleiche Länge wie ein Spritzenzylinder derart umfasst, dass eine Spritze in ein Spritzenaufnahmeloch in den Körper eingeführt und durch die Druckkraft festgehalten wird, die durch die fingerartigen Elemente zwischen einem Spritzenfingerflansch und einer Körperbasis aufgebracht wird.

12. Vorrichtung nach Anspruch 1 oder Verfahren nach Anspruch 2, bei der/dem das Gehäuse einen Körper (127) mit einem Spritzenaufnahmeschlitz (128) umfasst, der bemessen ist, um einen Spritzenzylinder aufzunehmen.

13. Vorrichtung nach Anspruch 1 oder Verfahren nach Anspruch 2, bei der/dem das Gehäuse einen puckartigen Körper mit einer Spritzenaufnahmeöffnung umfasst, so dass bei Verwendung eine Spritze in die Spritzenaufnahmeöffnung eingeführt und mit einem Daumen eines Benutzers auf einem Unterseitendaumengriff an Ort und Stelle gehalten wird.

## Revendications

1. Dispositif permettant d'entraîner des utilisateurs à mélanger correctement des composants pharmaceutiques, le dispositif comprenant :
un logement qui s'étend le long d'un axe longitudinal ;
une source de puissance (151) disposée dans le logement ;
un micro-dispositif de commande (154) disposé dans le logement et alimenté en puissance électrique par la source de puissance (151) ;
un dispositif de notification d'utilisateur (155) ; et
un accéléromètre (150) disposé dans le logement et connecté électriquement au micro-dispositif de commande (154) de sorte que le micro-dispositif de commande (154) est configuré pour détecter un mouvement et une orientation du logement et indiquer par l'intermédiaire du dispositif de notification d'utilisateur (155) si le mouvement ou l'orientation du logement qui est secoué pendant l'un parmi une administration de médicament ou un événement d'entraînement est suffisant par rapport à des seuils prédéterminés comportant la grandeur de la force appliquée pendant le secouage, l'orientation du logement et la durée d'un tel secouage, et dans lequel
le micro-dispositif de commande (154) est configuré pour détecter si le niveau de vigueur de secouage a diminué à un niveau en dessous du seuil préréglé et pour entrer dans un mode de pause pour permettre à l'utilisateur de redémarrer le secouage pendant l'un parmi une administration de médicament ou un événement d'entraînement.

2. Procédé pour diriger un utilisateur concernant une technique adéquate de mélange de médicament avec un dispositif d'entraînement ou un dispositif permettant de mélanger et aider à l'administration du médicament, qui comporte :
un logement qui s'étend le long d'un axe longitudinal ;
une source de puissance (151) disposée dans le logement ;
un micro-dispositif de commande (154) disposé dans le logement et alimenté en puissance électrique par la source de puissance (151) ;
un dispositif de notification d'utilisateur (155) ; et
un accéléromètre (150) disposé dans le logement et connecté électriquement au micro-dispositif de commande (154) pour détecter un mouvement et une orientation d'un tel mouvement,
le procédé comprenant les étapes consistant à :
déterminer à partir de l'accéléromètre (150) si la grandeur du mouvement et l'orientation du logement sont suffisantes par rapport à des seuils prédéterminés comportant la grandeur de la force appliquée pendant le secouage, l'orientation du logement et la durée d'un tel secouage ; et
annoncer par l'intermédiaire du dispositif de notification d'utilisateur (155) si le mouvement ou l'orientation du logement qui est secoué pendant l'un parmi une administration de médicament ou un événement d'entraînement respecte les seuils prédéterminés, et dans lequel
le micro-dispositif de commande (154) est configuré pour détecter si le niveau de vigueur de secouage a diminué à un niveau en dessous du seuil préréglé et pour entrer dans un mode de pause pour permettre à l'utilisateur de redémarrer le secouage pendant l'un parmi une administration de médicament ou un événement d'entraînement.

3. Dispositif selon la revendication 1 ou procédé selon la revendication 2, dans lequel le micro-dispositif de commande (154) est configuré pour détecter lorsque le secouage du logement a pris fin prématurément et pour entrer dans un mode de pause pour permettre à l'utilisateur de redémarrer le secouage pendant l'un parmi une administration de médicament ou un événement d'entraînement.

4. Dispositif selon la revendication 1 ou procédé selon la revendication 2, dans lequel le dispositif comporte en outre un interrupteur de démarrage (153) connecté électriquement au micro-dispositif de commande (154).

5. Dispositif selon la revendication 1 ou procédé selon la revendication 2, dans lequel l'accéléromètre (150) comprend un accéléromètre à 3 axes.

6. Dispositif selon la revendication 1 ou procédé selon la revendication 2, dans lequel l'accéléromètre (150) est configuré pour activer le micro-dispositif de commande (154) lors d'une détection de mouvements du logement pendant l'un parmi une administration de médicament ou un événement d'entraînement.

7. Dispositif selon la revendication 1 ou procédé selon la revendication 2, dans lequel le micro-dispositif de commande (154) est configuré pour régler un temporisateur et déterminer lorsqu'un temps maximum admissible après secouage du logement s'est écoulé pour avertir l'utilisateur de secouer le dispositif de nouveau pendant l'un parmi une administration de médicament ou un événement d'entraînement.

8. Dispositif selon la revendication 1 ou procédé selon la revendication 2, dans lequel le logement comprend un élément de cylindre de seringue (103) avec un support de doigts (100) et une extrémité et un bout de cylindre (104) espacés le long de l'axe longitudinal.

9. Dispositif selon la revendication 1 ou procédé selon la revendication 2, dans lequel le logement comprend un corps (109) avec une fente (111) dimensionnée pour accepter un cylindre de seringue (107) ou un flacon.

10. Dispositif selon la revendication 1 ou procédé selon la revendication 2, dans lequel le logement comprend un logement pourvu d'un compartiment et d'un couvercle pour recevoir une seringue entière.

11. Dispositif selon la revendication 1 ou procédé selon la revendication 2, dans lequel le logement comprend un corps allongé approximativement de la même longueur que le cylindre de seringue de telle sorte qu'une seringue est insérée dans un trou de réception de seringue dans le corps et retenue par la force de compression appliquée par les éléments de type doigt entre un support de doigts de seringue et une base de corps.

12. Dispositif selon la revendication 1 ou procédé selon la revendication 2, dans lequel le logement comprend un corps (127) avec une encoche de réception de seringue (128) dimensionnée pour recevoir un cylindre de seringue.

13. Dispositif selon la revendication 1 ou procédé selon la revendication 2, dans lequel le logement comprend un corps de type galet presseur avec un trou de réception de seringue de sorte qu'en cours d'utilisation une seringue est insérée dans le trou de réception de seringue et est maintenue en place avec le pouce d'un utilisateur sur un repose-pouce inférieur.
